# EUROPEAN PATENT SPECIFICATION

(11) **EP 4 419 056 B1**
(45) Date of publication and mention of the grant of the patent: **22.07.2026**
(21) Application number: 22812438.4
(22) Date of filing: 17.10.2022
(51) Int. Cl.: A61F 13/00, A61F 13/02, A61K 9/70, A61K 9/00

(54) **IMPROVED PATCHES AND METHODS FOR DELIVERING AN ACTIVE SUBSTANCE TO THE SKIN USING THE SAME**
VERBESSERTE PFLASTER UND VERFAHREN ZUR ABGABE EINES WIRKSTOFFS AN DIE HAUT DAMIT
TIMBRES AMÉLIORÉS ET PROCÉDÉS D'ADMINISTRATION D'UNE SUBSTANCE ACTIVE POUR LA PEAU LES UTILISANT

(30) Priority: 18.10.2021 US 202163256803 P; 21.12.2021 EP 21306887
(43) Date of publication of application: 28.08.2024
(60) Divisional application: 26186213.0
(73) Proprietor: DBV Technologies, 92320 Chatillon (FR)
(72) Inventor: VILLETTE, Jérôme, 92350 LE PLESSIS ROBINSON (FR); EHOUARN, Pascale, 91940 GOMETZ-LE-CHATEL (FR)
(74) Representative: Cabinet Becker et Associés
(86) International application number: PCT/EP2022/078796
(87) International publication number: WO 2023/066846

(56) References cited:
- EP-B1- 0 645 995
- EP-B1- 0 755 284
- WO-A2-2005/042054
- US-A1- 2006 105 029
- US-B2- 9 457 123

## Description

### TECHNICAL FIELD

The present invention relates, generally, to a patch for delivering an active substance to a subject using the skin, part of body and to the uses thereof, especially for providing immunotherapy by epicutaneous route.

### BACKGROUND

The assignee of the present application, DBV Technologies pioneered the use of patches containing a small amount of an allergen, such as biologics, or food proteins, as milk or peanut protein, electrostatically deposited on a substrate surface without an adjuvant, in a form that is water soluble. Such patches may be used for determining a subject's immune response to exposure to an allergen, or to conduct immunotherapy, such that a subject, by a course of wearing patches providing cutaneous exposure to an allergen over a period of weeks or months, builds up a tolerance to the allergen.

For example, U.S. Patent No. 7,635,488 describes a patch including a support that includes a central portion on which the allergen is coated as a solid and dry deposit, and a peripheral portion that may be adhesively coupled to the skin. When the peripheral portion is adhered to a subject's skin, the central portion of the support forms an occlusive or condensation chamber. The patch is worn for a specified period, during which perspiration from the skin subject condenses within the chamber and solubilizes the solid deposit of allergen coated on the internal surface of the central portion of the support, allowing the allergen to contact the skin and penetrate the epidermis. U.S. Patent No. 8,968,743 describes the use of such patches to deliver vaccines via an epicutaneous route. WO2005042054 describes another patch for delivering a substance to the body.

Such patch may further include a circular foam ring disposed on the backing and acting as a watertight joint between the backing of the patch and the skin of the subject whereby a hermetically closed chamber defined as occlusive chamber or condensation chamber, is obtained when the patch is applied on the skin.

In order to provide the desired effect to a subject, it is important that the patch remains on the subject's skin for a predetermined period of time. However, up to now, the adhesive properties of the patches may be insufficient and may lead to inconsistent adhesion of the patches on a subject's skin, and occasionally premature detachment.

It therefore would be desirable to provide patches that improve consistency in delivery of an active ingredient to the skin, handling application, and usability.

It also would be desirable to provide patches that facilitate, and enhance the consistency of, application of the patches to a subject.

It further would be desirable to provide patches having improved adhesion during the wearing time and reduce the risk of premature detachment from a subject's skin.

It still further would be desirable to provide patches having improved resistance to detachment caused by rubbing of a subject's garments against the patch, thereby further reducing the risk of dislodgment of the patch.

### SUMMARY OF THE INVENTION

The invention is defined by the claims.

It is an object of the present invention to provide a patch for delivering a biologically active substance to the skin of a subject, the patch comprising: a substrate; a biologically active substance disposed on the substrate; a foam ring having a periphery, a first skin-facing surface and a first layer of adhesive disposed on the first skin-facing surface, the foam ring disposed beneath the substrate to form, when applied to a subject's skin, an occlusive or condensation chamber in which the biologically active substance is located; a breathable film disposed over the substrate and having an exterior surface, a second skin-facing surface opposed to the exterior surface, and a second layer of adhesive disposed on the second skin-facing surface; and a paper applicator removably disposed on the exterior surface of the breathable film, the paper applicator having an aperture with an interior edge, the paper applicator configured for applying the patch to a subject's skin, wherein the breathable film covers the substrate and the foam ring, the aperture of the paper applicator is around and concentric with the foam ring and the paper applicator has an open notch, such as a V-shaped notch, to facilitate removal from the exterior surface of the breathable film.

The present invention also relates to a patch for delivering a biologically active substance to the skin of a subject, the patch comprising: (i) a biocompatible substrate; (ii) a biologically active substance disposed on the substrate; (iii) a biocompatible foam ring having a periphery, or outer edge, a skin-facing surface and a layer of biocompatible adhesive disposed on the first skin-facing surface, the foam ring disposed beneath the substrate to form, when applied to a subject's skin, a chamber in which the biologically active substance is located; (iv) a biocompatible breathable film having an exterior surface, a second skin-facing surface, and a layer of biocompatible adhesive disposed on the second skin-facing surface; and (v) a paper applicator removably disposed on the exterior surface of the breathable film, the paper applicator having an aperture with an interior edge, the paper applicator configured for applying the patch to a subject's skin, wherein the breathable film covers the substrate and the foam ring, and the aperture is around and concentric with the foam ring and provides a gap between the interior edge of the paper applicator and the periphery of the foam ring. Advantageously, the paper applicator has an open notch, such as a V-shaped notch, on the interior edge to facilitate removal from the exterior surface of the breathable film.

The present invention also relates to a patch for delivering a biologically active substance to the skin of a subject, the patch comprising: a substrate; a biologically active substance disposed on the substrate; a foam ring having a periphery, a first skin-facing surface and a first layer of biocompatible adhesive disposed on the first skin-facing surface, the foam ring disposed beneath the substrate to form, when applied to a subject's skin, an occlusive chamber; a breathable film having an exterior surface, a skin-patient facing surface, and a second layer of biocompatible adhesive disposed on the second skin-facing surface, and a paper applicator removably disposed on the exterior surface of the breathable film and configured for applying the patch to a subject's skin, the paper applicator having an opened edge, such as a V-shaped notch, to facilitate removal from the exterior surface of the breathable film.

According to the present invention, the open notch extends partially on the paper applicator, from an edge (interior edge or exterior edge) of the paper applicator. The open notch opens on an edge of the paper applicator and extends partially toward the opposite edge of said paper applicator. For instance, the open notch may be a U-shaped notch, a V-shaped notch, etc.

According to the present invention, the patch comprises successive layers that cooperate with each other to improve the adhesiveness of the patch on a subject's skin.

In view of the shortcomings observed with previously known patches, patches constructed in accordance with the present invention simplify manufacture and improve reproducibility of a patch for allergy testing or for providing delivery of active substance to the skin, such as the epidermis as in the case of epicutaneous immunotherapy. In particular, the inventive patches facilitate, and enhance the consistency of, application of the patches to a subject, while also demonstrating improved long-term adhesion over the wearing time, and reduced risk of premature detachment. By "long-term" it is meant over the course of the treatment, e.g., over a period of hours, a day, or even more than a day. Further, the inventive patches demonstrate improved resistance to detachment caused by rubbing of a subject's garments against the patch.

In accordance with the principles of the invention, the patches include multiple layers constructed having advantageously a round or rounded rectangular shape that reduce right angles that otherwise can lead to early loss of adhesion or provide focal spots that are lifted or curl up due to friction from rubbing garments. An inventive patch generally includes a substrate, to which a predetermined quantity of an active substance of interest is deposited in a solid form. A foam ring is coupled to the substrate around the active substance so as to provide a pre-formed occlusive or condensation chamber which may be adhered to skin in a watertight manner. The foam ring and the substrate may have substantially the same outer diameter.

The substrate may include any suitable material or combination of materials for supporting the active substance and that suitably may be coupled to foam ring, e.g., via adhesive on the adjoining surface of the foam ring. For instance, the substrate may include a polymer selected from cellulose plastics (such as cellulose acetate (CA) or cellulose propionate (CP)), polyvinyl chloride (PVC), polypropylene, polystyrene, polyurethane, polycarbonate, polyacrylic (such as poly(methyl methacrylate (PMMA)), polyester, polyethylene (PE), polyethylene terephthalate (PET), or a fluoropolymer (such as polytetrafluoroethylene (PTFE). The substrate may further include a conductive layer, e.g., a coating that includes metal layer or metal particles or metal oxide particles such as titanium oxide, aluminum, or gold, in order to facilitate deposit of the active substance by electrospray. In a particular embodiment, the substrate includes polyethylene terephthalate and a titanium layer coating on its skin-facing surface.

The foam ring may include any suitable combination of foam or polymer biocompatible material. Particularly, the foam or polymer material may be or include polyethylene vinyl acetate (PEVA), low-density polyethylene (LDPE), polypropylene (PP), polyethylene (PE), silicone, or mixture thereof.

Advantageously, the ratio between an outer diameter and an inner diameter of the foam ring may be about 1.20 to about 1.75, such as about 1.25, 1.30, 1.35, 1.40, 1.45, 1.50, 1.55, 1.60, 1.65, 1.70 or 1.75. The thickness of the foam ring is sufficient to create a condensation or occlusive chamber between the skin of the subject and the inner diameter of the foam ring when the patch is applied on the subject's skin.

The assembly is maintained on the skin by a breathable film that covers the substrate. More specifically, the breathable film extends beyond an exterior edge of the foam ring to adhere the patch to the subject's skin beyond the periphery of the foam ring. The dimensions of the breathable film are adapted to the dimension of the foam ring, so that the breathable film recovers entirely the foam ring and extends beyond the foam ring. For instance, the diameter or the greater dimension of the breathable film is at least 10% higher than the diameter of the foam ring, such as 20%, 30%, 40%, 50%, 55%, 60%, 65%, 70%, 75%, 80%, 85%, 90%, 95% or 100% higher. Particularly, the diameter or greater dimension of the breathable film may be about 65%, +/- 5% higher than the diameter of the foam ring.

Advantageously, the breathable film has circular shape or round angle corners.

The adhesive layer(s) on the foam ring and/or breathable film may include an acrylate adhesive, hydrocolloid adhesive, polyurethane adhesive, or silicone adhesive.

The patch may be provided in a ready-to-use form, having a release liner, in a protective role that is removed to expose an adhesive surface of the foam ring and the adhesive layer of the breathable film. The patch further is provided with a paper applicator associated with the non-adhesive surface of the breathable film. The paper applicator improves rigidity of the patch and facilitates handling, and then may be removed by a caregiver while adhering the patch onto the subject's skin. By "configured for applying the patch to a subject's skin" means that the paper applicator of the patch recovers at least partially the breathable film to confer temporarily a slight rigidity to the patch to facilitate its handling, and comprises means for allowing its peeling from the breathable film.

The paper applicator has a substantially ring shape (e.g., round or oblong) to fit the shape of the breathable film. The paper applicator has a central aperture with a round or oblong shape. The paper applicator is configured in such a way that the aperture of the paper applicator match with the foam ring.

The width of the paper applicator, corresponding to the dimension extending radially from the exterior edge to the interior edge of the paper applicator (i.e., the edge that borders the aperture) may be about 5% to about 20% of the greater dimension of said paper applicator, such as about 8%, 9%, 9.5%, 10%, 12%, 15%, 15.5%, 16%, 17%, 18%, 19%. That is to say that the greater dimension, or the diameter, of the aperture of the paper applicator may be about 80% to 95% the greater dimension of said paper applicator, such as about 81%, 82%, 83%, 84%, 84.5%, 85%, 88%, 90%, 90.5%, 91%, 92% the greater dimension of the paper applicator. For instance, the width of the paper applicator is about 8% to about 16% of the greater dimension of the paper applicator, such as about 9% to about 9.5%, or about 15% to about 15.5%.

The greater dimension of said aperture is strictly greater than the diameter of the foam ring (e.g. at least 105%, at least 110%, at least 125% or at least 150% of the external diameter of the foam), so that a portion of the breathable film, forming a crown around the foam ring (herein after a "gap") extends around and between the interior edge of the paper applicator and the periphery of the foam ring. Particularly, the width of the gap (i.e., the distance between the interior edge of the paper applicator and the periphery of the foam ring) may be equal to about 1/4 to 2/3 of the distance between the exterior edge of the breathable film and the periphery of the foam ring, for example between 1/3 and 1/2, or between 1/2 and 2/3 of the distance between the exterior edge of the breathable film and the periphery of the foam ring. The width of the gap may be constant all around the foam ring or the width of the gap may vary around the foam ring, to be greater on some parts and smaller on others. In a particular embodiment, the gap has a round shape. In another embodiment, the gap has an oblong shape.

The width of the paper applicator may be about 10% to about 200% the width of the gap between foam ring and paper applicator, e.g., about 20% to about 180% the width of the gap, or about 50% to about 150% the width of the gap, or about 80% to about 120% the width of the gap, or about 90% to about 110% the width of the gap, or about 100% the width of the gap. For instance, the width of the paper applicator is about 10% to about 95% the width of the gap, such as about 20% to about 95%, or about 50% to about 95%.

In a particular embodiment, the width of paper applicator covers approximately half of the distance by which breathable film extends beyond the outer edge of the foam ring.

The paper applicator may include any suitable material of combination of materials, such as siliconized paper (e.g., paper coated with silicone on one or both sides and optionally including a hydrophobic coating, such as PTFE).

According to the present invention, the paper applicator may have an open notch, such as a V-shaped notch, to facilitate removal from the exterior surface of the breathable film. The notch may be managed on the interior edge or on the exterior edge of the paper applicator. Particularly, the open notch may lead to the interior edge of the paper applicator and may be radially directed so as to extend from the internal diameter of the paper applicator partially towards its external diameter. Alternatively, the open notch may lead to the exterior edge of the paper applicator and may be radially directed so as to extend from the external diameter of the paper applicator partially towards its internal diameter.

In particular, the open notch is a V-shaped notch, which may lead either to the interior edge or to the exterior edge of the paper applicator, and is radially directed so as to extend from the internal diameter of the paper applicator partially towards its external diameter, or the reverse. The V-shaped notch may be formed with an angle α of about 45° +/-10°, to easily grab the edge of the notch. Furthermore, an apex of the V-shaped notch may be curved (e.g., radiused apex), to avoid sharp edges that might interfere with manufacturing.

The apex of the notch is aligned with one or several successive slits that extend radially, from the apex of the notch to the exterior edge (or interior edge) of the paper applicator. Advantageously, the slits do not extend entirely across the width of paper applicator, thereby ensuring that paper applicator cannot come free during manufacture. These slits may facilitate further tearing away of paper applicator once the patch adheres onto the subject's skin. The notch and optional slits allow to remove the paper applicator with reduced shear forces applied on the breathable film and/or foam ring.

In particular, the paper applicator comprises a plurality of aligned and discontinuous slits, which extend radially and successively from an edge of the paper applicator toward the open notch. Advantageously, one of said plurality of slits opens on the end, or apex, of the notch.

For instance, the paper applicator comprises a V-shaped notch and a plurality of aligned slits, wherein one slit of said plurality opens on the apex of the V-notch.

In particular, the paper applicator comprises an open notch, such as a V-shaped notch, and two successive slits. The open notch is radially directed on the paper applicator and the opened end of the notch leads on a first edge of the paper applicator. A first slit extends radially from the closed end, or apex, of the notch, partially toward the opposite edge of the paper applicator (i.e., second edge). A second slit extends radially between the first slit and the second edge. The first slit may open on the apex of the notch. Alternatively of in addition, the second slit may open on the second edge.

A distance between two successive slits depends on the number of slits, the dimensions of each slit, and/or on the distance between the apex of the notch and the opposite edge of the paper applicator. For instance, the open notch extends radially from an edge of the paper applicator, onto 40% to 60% of the width of the paper applicator. The paper applicator further comprises two successive slits. The first slit extends from the apex of the notch and has a length of about 10% to about 15% of the width of the paper applicator, and the second slit has a length of about 10% to about 15% of the width of the paper applicator. A non-cut part of the paper applicator extending between the two successive slots has a length of about 10% to about 25% of the width of the paper applicator.

Advantageously, the exterior edge of the paper applicator overlaps with the exterior edge of the breathable film.

Further in accordance with the principles of the invention, the release liner includes a slit that facilitates removal of the liner from the patch to expose the adhesive surfaces of the foam ring and breathable film substantially without distorting the film. Particularly, the slit may be asymmetrically located so as to be offset from the portion of the substrate that defines the condensation chamber. This may inhibit moisture from entering the condensation chamber via the slit. The slit may overlap the foam ring, so as to facilitate peeling the release liner off of the rest of the assembly.

In addition, in nonlimiting examples, the paper applicator is configured in the shape of a ring having a width of about one-half the width of the film that extends beyond the exterior edge of the foam ring. The paper applicator also may include a radially directed notch, having for instance a V shape, with paper not completely split, just cut on both side and ready to be manually broken, for facilitating peeling the paper applicator from the exterior surface of the breathable film once the patch is applied to the subject's skin. Advantageously, the paper applicator is configured so that it does not cause the patch to lift off the subject's skin when the applicator is being removed.

The slit on the release liner is advantageously located on a first half of the patch, whereas the open notch and optional slit(s) on the paper applicator is/are preferably located on a second opposite half of the patch. Alternatively or in addition, a longitudinal axis of the slit on the release liner extends perpendicularly to a longitudinal axis of the open notch on the paper applicator. Thereby, the removal of the release liner does not distort the part of paper applicator with the open notch. That limits the risks of uncontrolled and/or unwanted peeling of the paper applicator.

In a particular embodiment, the patch is round shaped or rectangular, and
- the diameter or greater side of the breathable film is between 40 mm +/-5mm and 55 mm+/-5mm, preferably between 45 mm +/-1mm and 50 mm +/-1mm,
- the external diameter of the foam ring is between 20 mm +/-5mm and 35 mm+/-5mm, preferably between 25 mm +/-1mm and 30 mm +/- 1mm,
- the internal diameter of the foam ring (corresponding to the diameter of the occlusive chamber) is between 15 mm +/-5mm and 25 mm+/-5mm, preferably between 18 mm +/-1mm and 20 mm +/- 1mm,
- the diameter of the substrate is substantially equal to the external diameter of the foam ring,
- the exterior edge of the paper applicator overlaps with the exterior edge of the breathable film, and
- the diameter or greater dimension of the aperture of the paper applicator is between 30 mm+/-5mm and 52mm +/-5mm, such as between 32 mm+/-1mm and 44 mm+/-1mm or between 38 mm+/-1mm and 52 mm+/-1mm.

The paper applicator may have an open notch, such as a V-shaped notch, radially directed from the interior edge of the paper applicator partially towards its internal diameter with one or two slits extending radially from the apex of the V-shaped notch toward the exterior edge of the paper applicator.

The active substance may be disposed on the substrate in any suitable manner. In one nonlimiting example, the biologically active substance may be bound by electrostatic forces to the substrate. However, it will be appreciated that the biologically active substance may be applied in any suitable manner, e.g., spraying and drying, or using an adhesive. In some examples, the biologically active substance is selected from an allergen, an antigen or a biologically active polypeptide (or peptide). The substance may include particles.

Preferably, the patch is typically prepared and/or conserved under vacuum, or under an inert atmosphere, such as dry nitrogen, in a single-use tear-open pouch.

### BRIEF DESCRIPTION OF THE DRAWINGS

The above and other advantages of the present invention will be apparent upon consideration of the following detailed description, taken in conjunction with the accompanying drawings, in which like reference characters refer to like parts throughout, and in which:
FIGS. 1A and 1B are, respectively, a plan view and cross-sectional view of an embodiment of reference patch employed in clinical trials of epicutaneous immunotherapy.
FIGS. 2A and 2B are, respectively, a perspective view and an exploded perspective view of the patch of FIGS. 1A and 1B;
FIGS. 3A and 3B are, respectively, a plan view of a patch according to an embodiment of the present invention and a plan view of the notch detail A shown in FIG. 3A;
FIG. 4 is an exploded perspective view of the patch of FIGS. 3A and 3B;
FIGS. 5A and 5B are, respectively, a plan view of a patch according to another embodiment of the present invention and a plan view of the notch detail B shown in FIG. 5A; and
FIG. 6 is an exploded perspective view of the patch of FIGS. 5A and 5B.

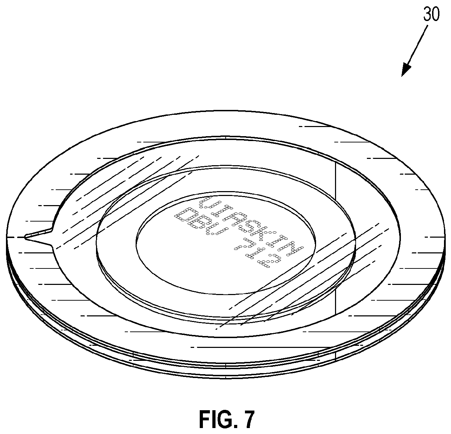

FIG. 7 is a perspective view of the patch of FIGS. 3A-3B and 4.

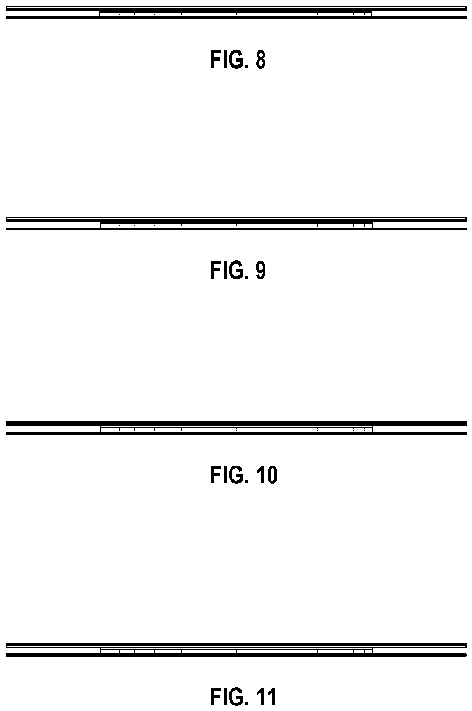

FIGS. 8, 9, 10, and 11 are side views of the patch of FIG. 7.

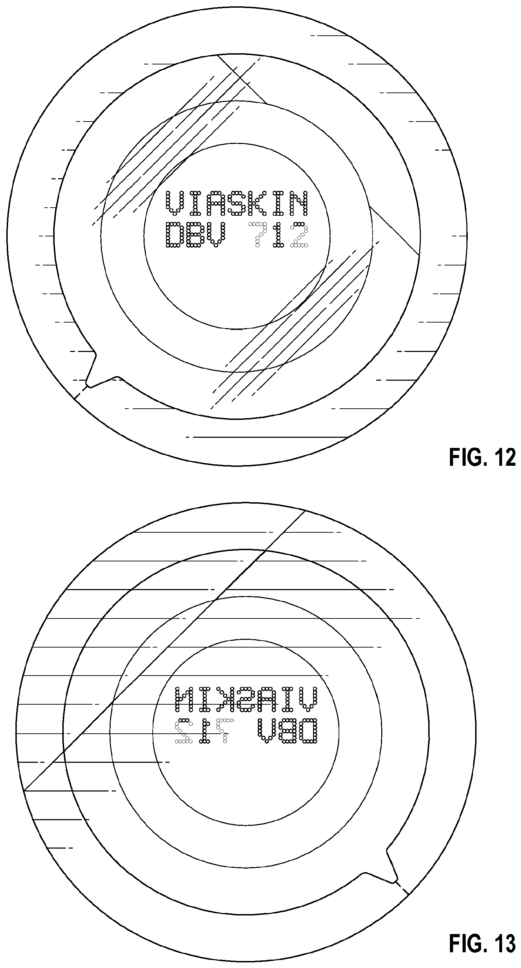

FIG. 12 is a top view of the patch of FIG. 7.
FIG. 13 is a rear view of the patch of FIG. 7.

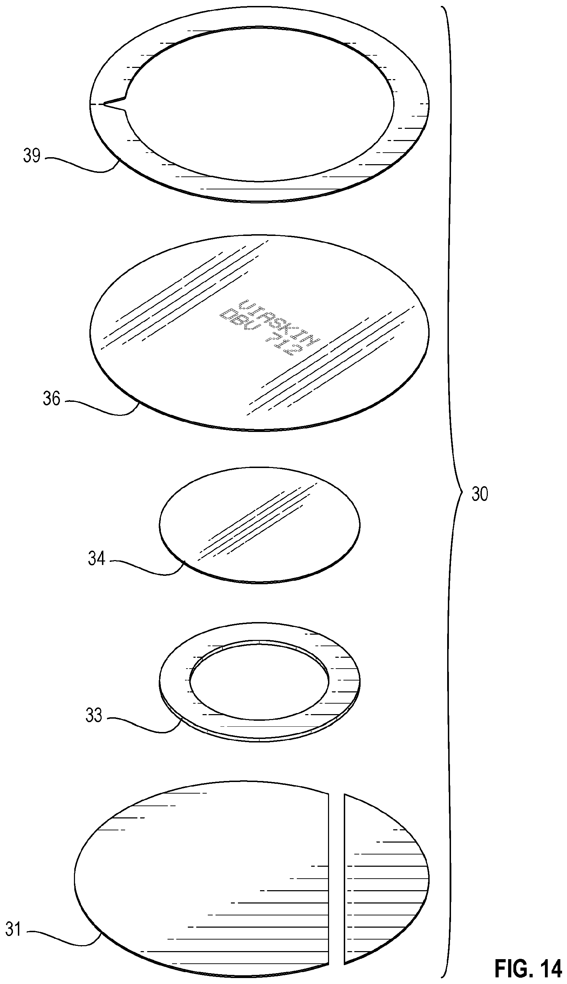

FIG. 14 is an exploded perspective view of the patch of FIG. 7.

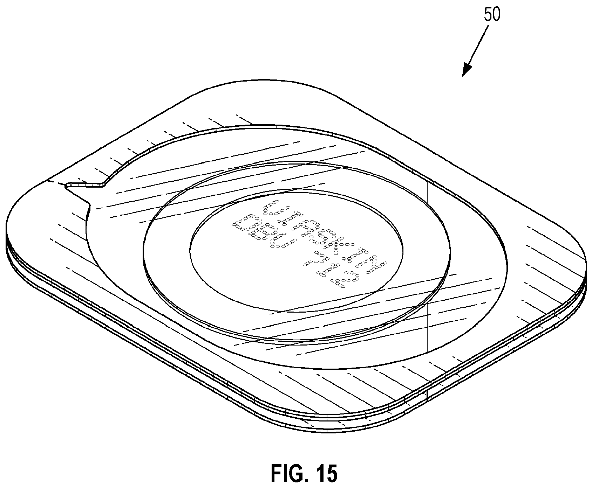

FIG. 15 is a perspective view of the patch of FIGS. 5A-5B and 6.

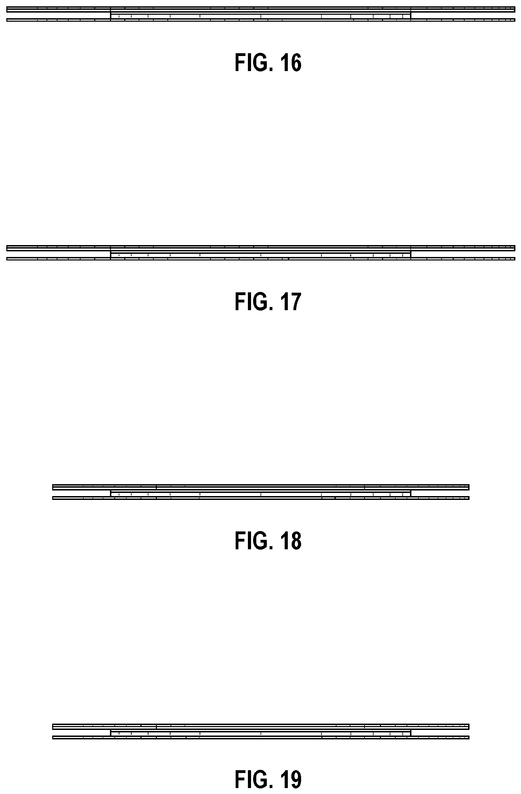

FIGS. 16, 17, 18, and 19 are side views of the patch of FIG. 15.

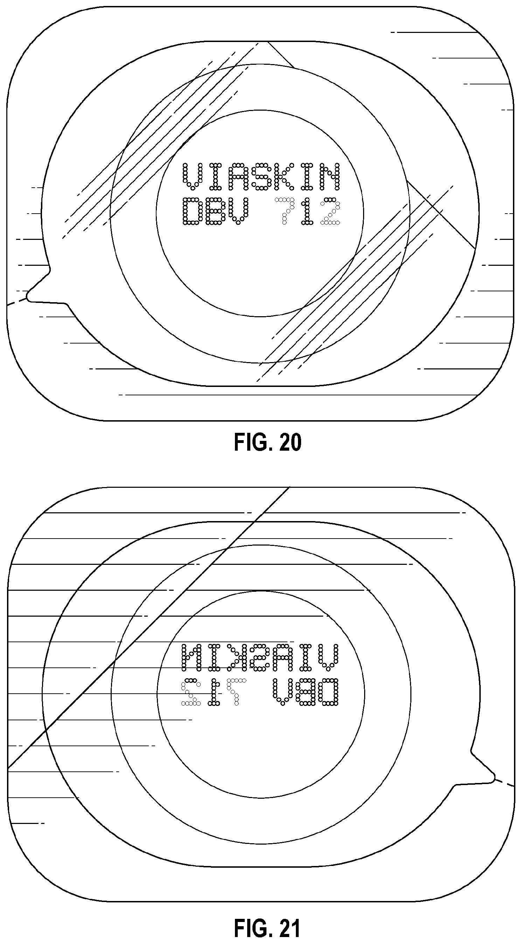

FIG. 20 is a top view of the patch of FIG. 15.
FIG. 21 is a rear view of the patch of FIG. 15.

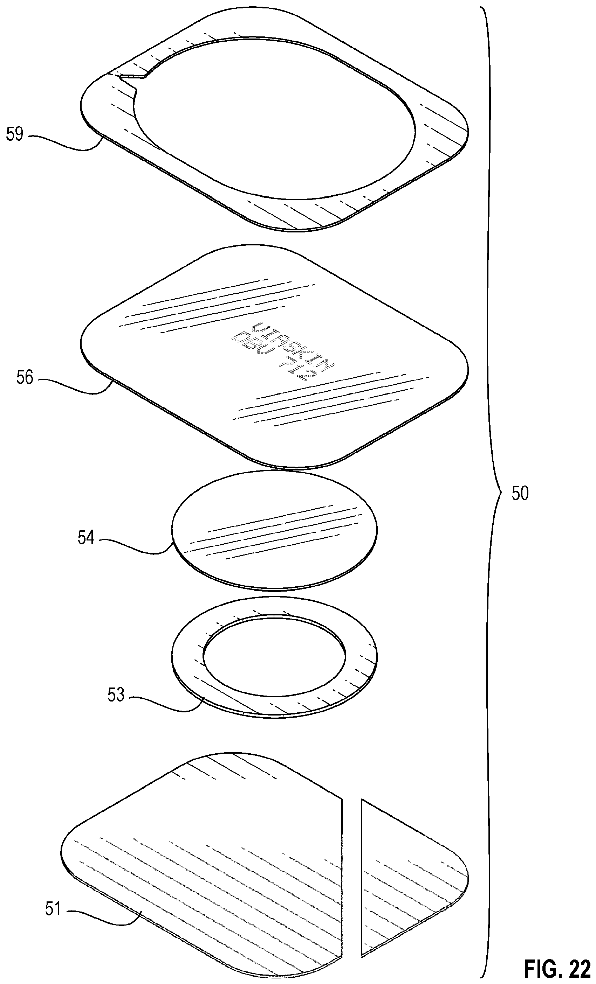

FIG. 22 is an exploded perspective view of the patch of FIG. 15.
FIGS. 23-25 schematically illustrate intermediate operations during application of a reference patch (previously conceived by the Applicant). Figures 23 and 24 show the removal of the release liner from the edge of the breathable film and figure 25 illustrates the debonding front lines and their concentration near the condensation chamber.
FIGS. 26-28 schematically illustrate intermediate operations during application of a patch of the present invention. Figure 26 illustrates an embodiment of the patch of the present invention, and figures 27-28 show the removal of the release liner
FIG. 29 illustrates example patch designs according to the present invention.

### DETAILED DESCRIPTION

The patches constructed in accordance with the principles of the present invention provide improvements upon the patches described in commonly assigned U.S. Patent Nos. 7,635,488 and 8,968,743. As discussed in this specification, the improvements simplify manufacturability; improve reproducibility of results for allergy testing or providing epicutaneous immunotherapy ("EPIT"); facilitate and enhance the consistency of application of the patches to a subject and/or demonstrate improved long term adhesion during the treatment, and/or reduce the risk of premature detachment, including by providing improved resistance to detachment caused by rubbing of a subject's garments against the patch, while inhibiting an unpleasant level of pain at removal.

As used herein, the term "epicutaneous" is intended to mean applied to the skin. Epicutaneous administration typically comprises skin application under condition sufficient to allow penetration or diffusion of the compound at least into the superficial layer(s) of the skin, such as the stratum corneum and optionally one or more other epidermis layers.

As used herein, the term "biocompatible" is intended to mean not harmful to living tissue.

As used herein, the term "breathable" is intended to mean being water vapor permeable, e.g., having a non-zero water vapor transmission rate.

As used herein, the term "ring" is intended to mean a structure that encloses an area. A ring may have any suitable shape, e.g., may be substantially circular, substantially oblong, substantially rounded square, substantially rounded rectangular, or the like. A ring may have an outer, or exterior, edge and an inner, or interior, edge. The shapes of the outer edge and the inner edge may be similar to one another or may be different than one another. By "rounded" it is meant having a radius of curvature adapted to the round and rectangular patch shapes, as opposed to a sharp perpendicular angle corner. For example, a rounded shape may have a radius of curvature of at least about 10% of the patch dimension, or at least about 20% of the patch dimension, or at least about 50% of the patch dimension, illustratively a radius of curvature of at least about 5 mm, at least about 9 mm (e.g., for a rounded rectangular patch having a size of about 36 mm by about 44 mm), or at least about 22 mm (for a circular patch having a diameter of about 44 mm).

As used herein, the terms "substantially," "approximately," "around" and "about" used throughout this specification are used to describe and account for small changes or fluctuations, such as due to variations in dimension. For example, they may refer to less than or equal to ±10%, such as less than or equal to ±5%, such as less than or equal to ±2%, such as less than or equal to ±1%, such as less than or equal to ±0.5%, such as less than or equal to ±0.2%, such as less than or equal to ±0.1%, such as less than or equal to ±0.05%.

As used in this specification, the terms "biologically active substance," "active substance," "active ingredient," and the like are intended to mean a substance for diagnostic, therapeutic, or preventive purposes. The biologically active substance or ingredient may be as an allergen, an antigen, a small molecule drug, or any therapeutic agent of interest such as a hormone, an aptamer, an antibody, or the like. The biologically active substance may be a peptide, a polypeptide, including recombinant protein, an oligo- or a polysaccharide, a nucleic acid, or the like. The biologically active substance or ingredient may be an isolated molecule, as well as an extract, such as a protein extract. The biologically active substance or ingredient may be combined with any appropriate excipient. In a preferred embodiment, the biologically active substance is selected from an allergen, an antigen or a biologically active polypeptide (or peptide). For instance, the biologically active substance may be a food allergen, such as an allergen from egg, milk, or peanut, e.g. a protein extract obtained from such food, or an aeroallergen such an allergen from dust mites or pollens. The patch of the invention can be used for providing desensitization, namely increasing the tolerance against a given allergen, in a subject allergic to said allergen. In other embodiments, the biologically active substance may be from a pathogen such as a pathogenic bacterium or virus, and the patch is used in a method for providing vaccination against such a pathogen.

As used herein, the terms "open notch" refer to a not fully cut notch, which comprises an end opening on an edge (interior or exterior) of the paper applicator. The open notch extends from an end of the paper applicator partially toward the opposite edge of said paper applicator. In other words, the open notch does not extend throughout the entire width of the paper applicator. The open notch may have any shape showing an opened end, such as a U-shape, a V-shape, etc.

As will be discussed below, in some examples, the biologically active substance is "dry layer coated." For example, the substance may either be available as a dry layer or be transformed or treated to become a dry layer (e.g., through lyophilization, heating and spraying, micronization, etc.) For example, the substance may be in a solid state, typically in the form of aggregates, or agglomerated particles. In other examples, the active substance may be in a liquid or semi-solid (e.g., gel, jelly, foam, or paste) form. The active substance may be applied to the relevant non-adhesive portion of the substrate in any suitable manner and may be dried there to form a solid or may be maintained in a liquid or semi-solid form. In some examples, the active substance is not mixed with any adhesive, excipient, or other ingredient. In some other embodiments, the active substance is present in combination with one or several pharmaceutically acceptable excipients.

The substance may be available naturally or commercially in the form of a dry layer, i.e. in the form of individualized particles, such that it does not require any particular treatment or transformation, other than perhaps decreasing the size of the particles thereof, if necessary.

The substance may alternatively be available in a large solid form. In this case, it may be first preferred to reduce the substance to individualized particles, optionally after transformation aimed at ensuring its conservation without denaturation.

In a further alternative, the natural substance may be in liquid form. In such a situation, the substance may be lyophilized, so as obtain a dry layer coated form. The dry layer coated form may be obtained by known techniques such as, for example, lyophilization (freezing and sublimation under vacuum) or heating and spraying, the choice of these techniques, in particular the degree of micronization, being left to the assessment of those skilled in the art as a function of the physicochemical characteristics of the substance under consideration.

To provide for conservation of the patch in its packaging, and in particular to avoid modification of the substance by ambient air, the particles typically undergo a particular treatment, such as lyophilization, and more particularly any treatment known to those skilled in the art.

Within the context of the present invention, the term "electrostatic force" generally designates any non-covalent force involving electric charges. More specifically, this term refers to two kinds of forces, that may act separately or together: Coulombic forces between space charges of the surface and charged particles and/or Van der Waals forces between the space charges of the surface and the particles. The intensity of the force between a surface and a particle can be enhanced or lowered by the presence of a thin water film due to the presence of moisture. Generally, the patch is made and kept in a dry place. The moisture preferably is low enough to allow the active ingredient to be conserved until the patch is applied to the skin. The moisture rate may be regulated to achieve suitable adhesion forces until the patch is applied to the skin.

As used herein, the expression "substrate" denotes any support made of a biocompatible material suitable for supporting an active substance. In nonlimiting examples, the substrate is electrically conductive so as to facilitate deposition of the active substance using an electrospray process in which an electric voltage is applied between the substrate and an electrospray nozzle from which a liquid including the active substance is dispensed and guided to the substrate via electric field. After the deposition process, electrostatic forces between the substrate and the active substance may retain the active substance at the substrate until the patch is applied to the skin to form an occlusive or condensation chamber in a manner such as described elsewhere herein. Illustratively, the substrate may include metallic particles.

The patch of the present invention may be used to deliver or expose a substance through or to the skin of a mammalian subject. As discussed above, the substance is directly or indirectly bound to a surface of the patch through electrostatic forces. In particular, the inventive patch includes a support or substrate to which a (dry layer coated) biologically active substance is directly or indirectly bound through electrostatic forces. The substrate is associated with a foam ring spacer that, together with the substrate, forms a chamber when the patch is applied to the skin a subject, thereby allowing a release of the biologically active substance through moistening.

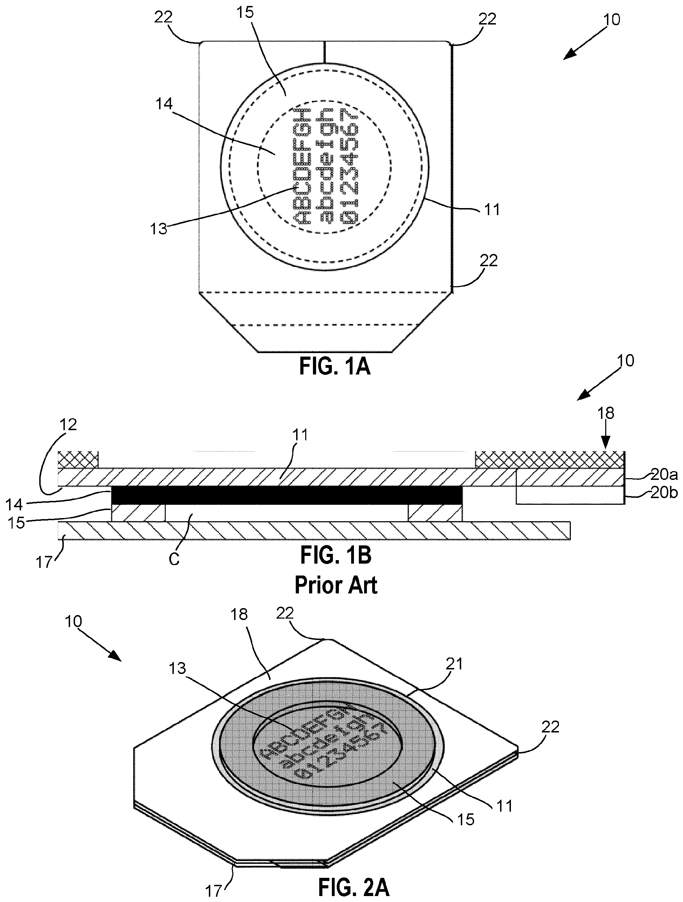

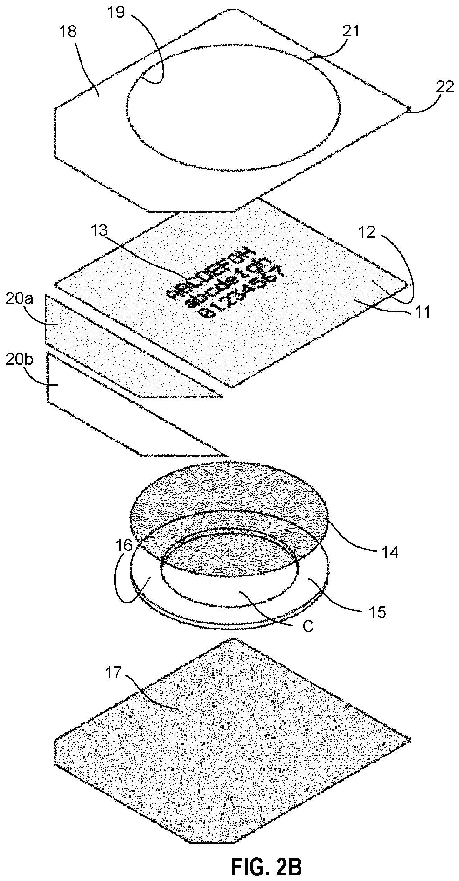

Referring to FIGS. 1A, 1B, 2A, and 2B, the details of the construction of reference patches previously conceived by the Applicant are provided as context to illuminate the improvements described and claimed in this specification.

In FIG. 1A, a plan view of reference patch 10 is described as it appears when removed from its storage pouch, looking onto the surface that faces away from the subject's skin. FIG. 1B depicts a cross-sectional view of patch 10, with the layers separated from one another for clarity. Patch 10 includes breathable film 11 having biocompatible adhesive layer 12 disposed on its skin-facing surface, label 13, substrate 14, foam ring 15 having adhesive lower surface 16, and release liner 17. Release liner 17 engages adhesive layer 12 of breathable film 11 and adhesive lower surface 16 of foam ring 15 and is removed prior to application of patch 10 on a subject's skin. The biologically active substance is bound by electrostatic forces to the skin-facing surface of substrate 14. Foam ring 15 supports the biologically active substance on substrate 14 out of contact with the subject's skin, so that when applied to the subject's skin, foam ring 15 and substrate 14 form chamber C that collects moisture emanating from the skin. The collected moisture hydrates the skin while enabling the solid active substance particles to solubilize and penetrate the patient's superficial skin (substratum corneum).

Referring now also to FIGS. 2A-2B, patch 10 includes paper applicator 18 that is lightly adhered to the outward-facing surface of breathable film 11. Paper applicator 18 acts as a protective layer for breathable film 11 and facilitates for the caregiver to firmly adhere patch 10 to the subject's skin. Paper applicator 18 includes aperture 19 that surrounds the periphery of substrate 14 and foam ring 15. In this way, it is intended that the caregiver will press the foam ring 15 to adhere the ring to the skin, but will not inadvertently press down on the center of substrate 14 and collapse the biologically active substance into direct contact with the subject's skin. Aperture 19 closely approximates the periphery of substrate 14 and foam ring 15. As described below, during testing, it was discovered that the high degree of coverage of paper applicator 18 on the exterior surface of breathable film 11 unintentionally imposes greater tension on breathable film 11 when paper applicator 18 is subsequently removed. Furthermore, the inventors found that instead providing the surface of the paper covering breathable film (ratio surface paper vs breathable film) of around 50% to 90%, and including a shape adapted and V-shaped notch instead of the slit of the design of FIGS. 2A-2B, improved the conformability of the breathable film during patch application on skin as compared to the design of FIGS. 1A-1B and 2A-2B.

Further with respect to FIGS. 1A-1B and 2A-2B, patch 10 includes removable tabs 20a and 20b for gripping patch 10 when release liner 17 is removed. As further determined during subsequent testing carried out at the direction of the inventors, tabs 20a and 20b increase the effort to manipulate patch 10 during preparation for placement on a subject's skin, while also increasing device complexity and reducing manufacturability. Furthermore, it was demonstrated that the shear forces applied when tearing off the tabs promoted detachment of the patch edge and corner. As described below, the improved patches of the present invention eliminate removable tabs 20a and 20b, thereby reducing manufacturing effort, and improving handling and reproducibility of patch application.

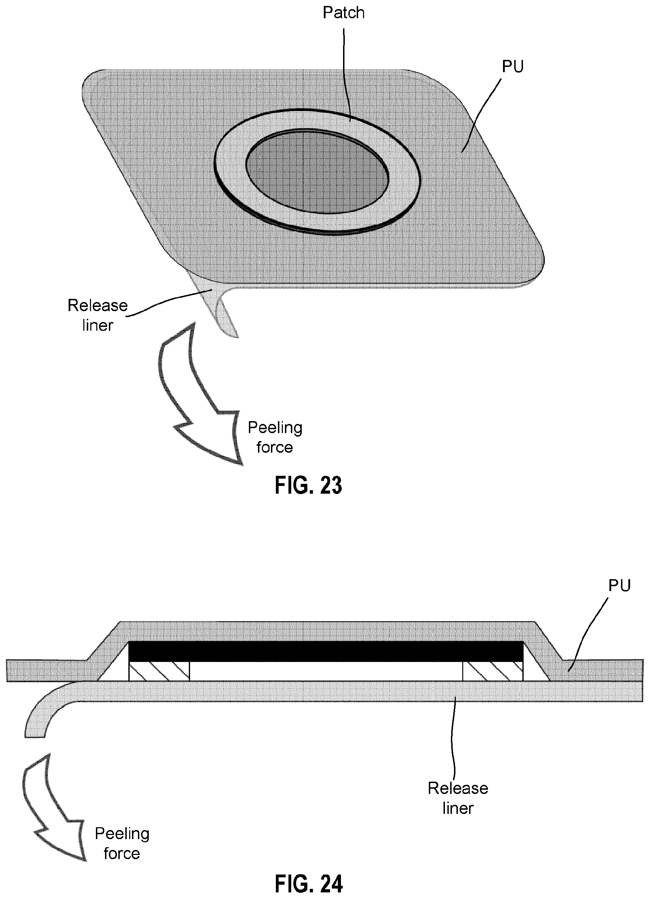

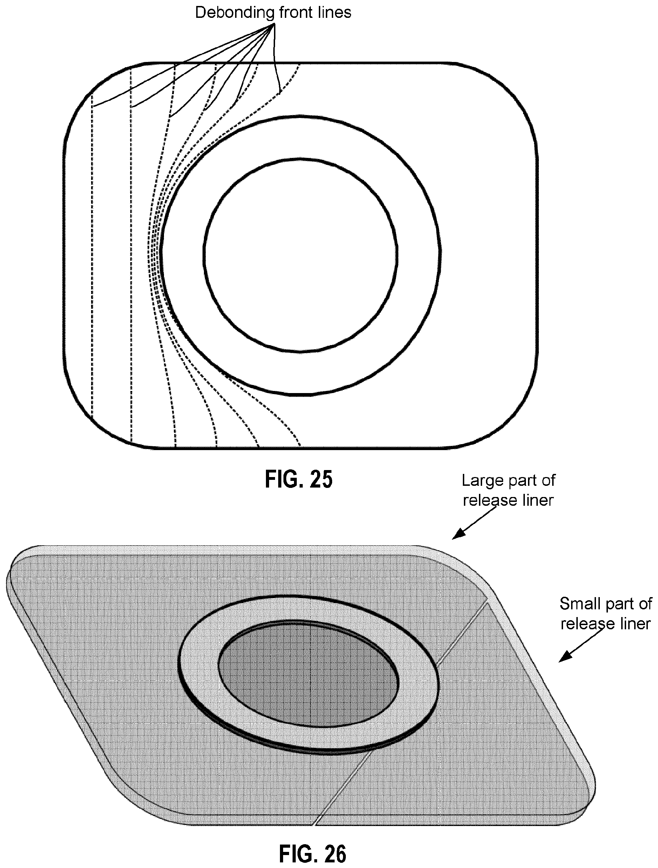

Fracture kinetic in the debonding of an adhesive from a surface is a well-known mechanism. Basically, in a homogeneous medium, the debonding front is orthogonal to the pulling direction and the peeling force is proportional to the length of debonding front. In patches such as described herein, debonding may occur for a weakly adherent layer such as a release liner (releasable layer) protecting for instance a wound dressing. For example, FIGS. 23-25 schematically illustrate intermediate operations during application of the present patches (i.e., the reference patches). One issue in the present patch system debonding is that it contains two regions of different adhesiveness and rigidity (see FIG. 23). The foam ring forming the patch chamber is more loaded in adhesive mass and stiff, while the PU adhesive (part of the dressing) is soft and less loaded with adhesive mass. Therefore, when starting debonding of the patch from its release liner by one of its border edges (FIG. 24), the debonding front propagates straight until it reaches the ring edge (FIG. 25). Therefore, the peeling force may abruptly induce large and concentrated strain, resulting in heavy distortion (wrinkles) and mechanical damage in the adhesive soft PU layer. The reference patch suffered from this issue, when peeling off the patch form release liner using the peeling tab.

Peeling tabs are commonly used in wound dressings, and can be made of a releasable sheet of paper covering the tacky adhesive of the dressing (see for instance EP0051935A2). The peeling tab may include a handle to apply the dressing on the skin. In such design the release liner is made from a single peeling part covering the whole adhesive of the dressing, including or excluding the peeling tab area.

In other designs, the release liner can be made in two parts, the first part disposed over one side of the dressing and the second part disposed over the remainder of the dressing. The first part may be adapted to provide handles whereby second parts can be grasped and peeled away from the adhesive (see for instance GB2128479A).

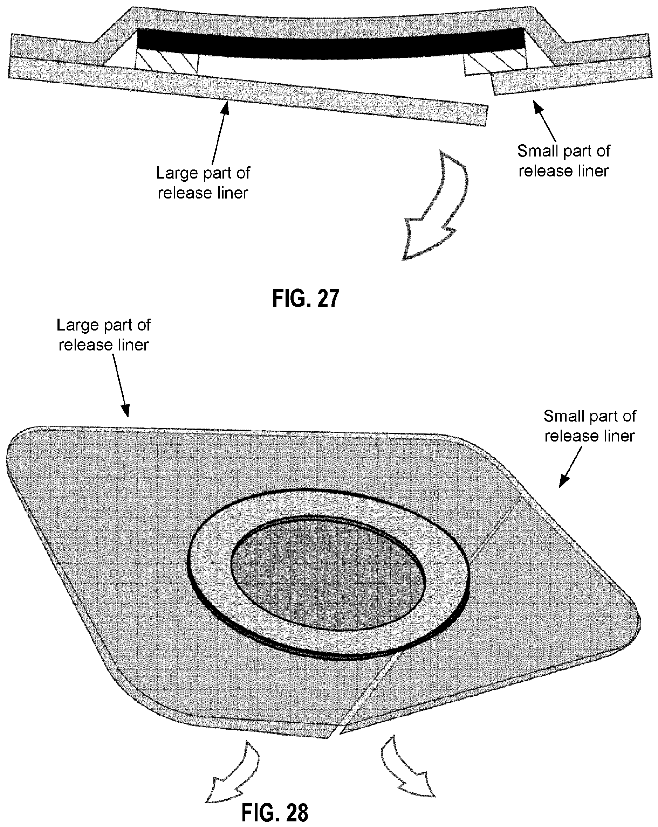

In the patch of the invention, the parting line in the release liner may not pass through or across the chamber, so that the chamber may maintain its integrity and airtightness. For example, in the patch of the present invention (FIGS. 26 to 28), the parting line separating the release liner may be located on the foam ring forming the patch chamber (FIG. 26). As such, when bending the patch, the large part can be grasped and easily peeled away (FIGS. 27 and 28). The peeling force is therefore directly exerted on the ring adhesive, not leading to strain in the PU adhesive layer.

The remaining part of the release liner still attached to the foam ring, forming a stiff handle, allowing placement on the skin without significant wrinkling of the dressing. After placement on the skin, when the major adhesive area is contacting the skin, the remaining part of the release liner can be peeled away.

Referring still to FIGS. 2A and 2B, paper applicator 18 includes slit 21 designed to assist in removal of paper applicator 18 once patch 10 is adhered firmly to the subject's skin. In particular, slit 21 was designed to facilitate peeling paper applicator 18 away from the breathable film 11. As observed during initial clinical testing, however, slit 21 proved difficult to employ, and resultantly contributed to excessive tension being applied to breathable film 11 during removal of paper applicator 18. As will be further observed in FIGS. 1A, 2A and 2B, patch 10 includes corners 22 that, while radiused, substantially form right angles. As determined during initial clinical testing and subsequent laboratory testing, such as described in the Working Examples below these substantially right-angle corners contribute to premature loss of adhesion between breathable film 11 and the subject's skin, as well as providing focal points for rubbing friction between patch 10 and the subject's overlying garments. Furthermore, the V-shaped notch of the present patches may be radially directed; as such, the forces applied to remove the present paper applicator are in the opposite direction as the adhesion forces, are of a lower magnitude, substantially do not deform the present breathable film, and limit peel off forces applied to the present breathable film.

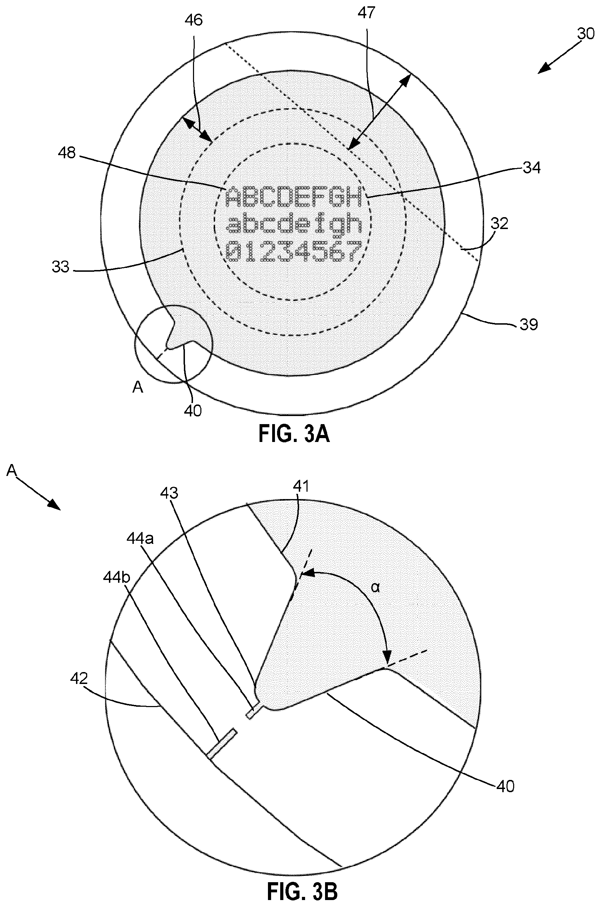

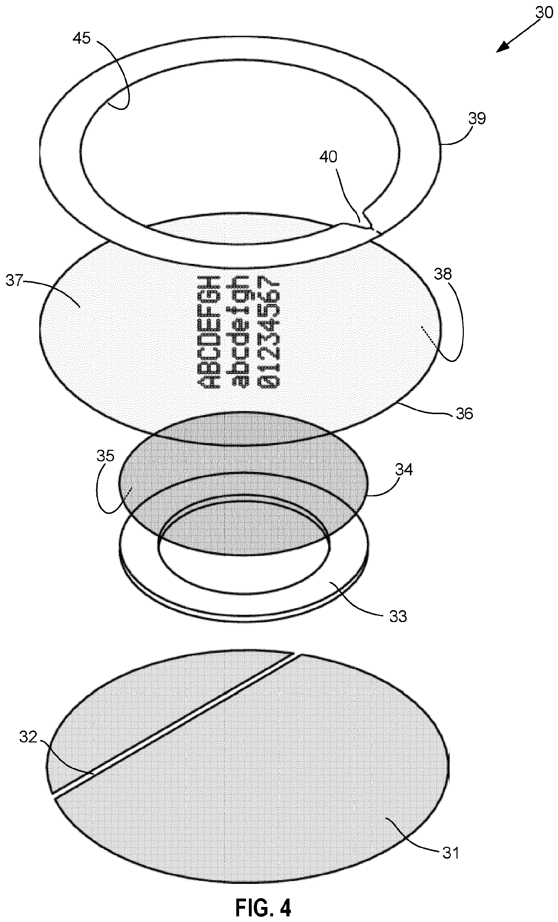

Referring now to FIGS. 3A, 3B and 4, an embodiment of the patch constructed in accordance with the principles of the present invention is described. The construction of patch 30, while having similar components to patch 10 of FIGS. 1A-1B and 2A-2B, is constructed based on extensive in-vitro testing to improve adherence to a subject's skin, improve handling, manufacturability, and reproducibility of result, and to reduce the risk of premature detachment.

In the example illustrated in FIGS. 3A, 3B, and 4, patch 30 includes: release liner 31 having slit 32; foam ring 33 having a biocompatible adhesive layer on a first skin-facing surface disposed in contact with release liner 31; substrate 34 that holds a biologically active substance on its skin-facing lower surface 35; breathable film 36 having exterior surface 37 and layer 38 of biocompatible adhesive disposed on its skin-facing surface; and paper applicator 39 disposed on exterior surface 37 of breathable film 36. It will be appreciated that all components of the present patches are biocompatible, even if not specifically described as such.

Foam ring 33 may include any suitable combination of foam or polymer biocompatible material suitable to space the substrate apart from the skin to define a chamber, and any suitable medical grade pressure sensitive adhesive(s) on its upper and lower surfaces. Preferably, foam ring 33 and the adhesive(s) thereon are of sufficient strength and flexibility so as to maintain hermeticity of the occlusive or condensation chamber when applied to the skin, even if the skin flexes or stretches. In nonlimiting examples, the foam or polymer material may be or include polyethylene vinyl acetate (PEVA), low-density polyethylene (LDPE), polypropylene (PP), polyethylene (PE), or silicone. In nonlimiting examples, the adhesive on the upper and/or lower surfaces of foam ring 33 (and in other components of patch 30) may include an acrylate adhesive, hydrocolloid adhesive, polyurethane adhesive, or soft silicone adhesive. Foam ring 33 may have any suitable shape and dimensions to support substrate 34 above a subject's skin, and to create a chamber, when applied to a subject. For example, foam ring 33 may have an external diameter between about 10 mm and about 100 mm, e.g., between about 20 mm and about 50 mm, or between about 20 mm and about 30 mm. Additionally, or alternatively, foam ring 33 may have an internal diameter that is at least about 1 mm less than any of the above external diameters (that is, the width of foam ring 33 may be at least about 1 mm). Particularly, the internal diameter of foam ring 33 may be about 2 mm to 50 mm less than the external diameter, or may be about 2 mm to 20 mm less than the external diameter, or may be about 5 mm to 15 mm less than the external diameter. In some examples, the ratio between the outer and inner diameters of the foam ring may be about 1.25 to about 1.5 (e.g., about 26 mm outer diameter to about 18 mm inner diameter, providing a width of the foam ring of about 3 mm to about 4 mm). Foam ring 33 may have any suitable thickness, e.g., about 0.1 mm to about 1 mm, e.g., about 0.5 mm to about 0.9 mm. In one nonlimiting example, foam ring 33 preferably has an external diameter of 26 mm +/- 5 mm, an internal diameter of about 18 mm, +/- 5mm and a thickness of about 0.5 mm, +/-0.2mm. Foam ring 33 and substrate 34 may have substantially the same outer diameter as one another. For example, these components may be assembled to one another and then die cut together.

Paper applicator 39 may include any suitable material of combination of materials, such as siliconized paper (e.g., paper coated with silicone on one or both sides and optionally including a hydrophobic coating, such as PTFE). In accordance with one aspect of the invention, paper applicator 39 has a ring shape, with V-shaped notch 40 that may be radially directed so as to extend from the internal diameter 41 partially towards the exterior diameter 42. As shown in FIG. 3B, corresponding to detail A in FIG. 3A, V-shaped notch 40 preferably has radiused apex 43 that is aligned with at least one slit, e.g., slits 44a and 44b, to facilitate tearing away of paper applicator 39 once patch 30 is firmly pressed onto the subject's skin. In addition, paper applicator 39 preferably includes central aperture 45 having an internal diameter that is at least about 1 mm greater than the external diameter of foam ring 33, so as to provide a gap of at least about 1 mm between the foam ring and the paper applicator. The internal diameter of paper applicator 39 may, for example, be about 1 mm to about 20 mm greater than the external diameter of foam ring 33, or about 2 mm to about 10 mm greater than the external diameter of foam ring 33, or about 3 mm to about 5 mm greater than the external diameter of foam ring 33. Paper applicator 39 may have any suitable external diameter that is at least 1 mm greater than the internal diameter of paper applicator 39 (that is, paper applicator 39 may have a width of at least 1 mm). For example, paper applicator 39 may have a width of about 1 mm to about 20 mm, or about 2 mm to about 10 mm, or about 3 mm to about 5 mm. In some example, the width of paper applicator 39 is about 10% to about 95% that of gap 46, e.g., about 20% to about 95% that of gap 46, or about 50% to about 95% that of gap 46. In one nonlimiting example, the width of paper applicator 39 is chosen to cover approximately half of the distance by which breathable film 36 extends beyond the outer edge of foam ring 33 (that is, to have approximately the same width as gap 46), and to have a minimal width of 3 mm to provide sufficient stiffness to facilitate handling. In one nonlimiting example, paper applicator 39 may have an inner diameter, corresponding to the diameter of the aperture, of about 30 to 40 mm (e.g., about 35 mm) and a width of about 2-4 mm (e.g., about 3 mm), thereby providing gap 46 (see FIG. 3A) of about 4 to 5 mm (e.g., about 4.5 mm) to the periphery of foam ring 33. As explained below, the width of paper applicator 39 and/or the size of gap 46 may contribute to better control of placing patch 30 during initial placement, and better longer-term adhesion, with improved manufacturability and uniformity of results compared to patch 10 such as described in the Working Examples below.

In particular, it had been observed during initial clinical trials of patch 10 of FIGS. 1A-1B and 2A-2B that after a relatively short period, e.g., significantly less than the 24 hours treatment period, the patch would begin losing adhesion to the subject's skin such as described in the Working Examples below. This loss of adhesion is believed to have largely resulted from the removal of paper applicator 18, which is peeled off the exterior surface of film 11 immediately after patch 10 is applied. Additionally, the removal of the peel tab is believed to have induced shear forces on the edge and corner of the patch. A loss of adhesion was believed to arise from different causes, which patch 30 of the present invention addresses. First, in patch 10, inner diameter of aperture 19 of paper applicator 18 closely approximates the periphery of foam ring 15. Second, slit 21 in paper applicator 18 was observed to be difficult to manipulate. These two features of patch 10 were observed during testing to require greater force to lift paper applicator 18 away from the exterior surface of film 11, and to require greater manipulation to grasp and open slit 21. Together, these features of patch 10 contributed to the application of greater tensile force being applied to film 11 after initial placement of patch 10 and degraded both the initial and long-term adhesion.

In accordance with the principles of the present invention, as determined by in vitro testing such as described in the Working Examples below, applicant determined that it was preferable to reduce the width of paper applicator 39 in the patch 30 to thereby reduce the coverage of the paper applicator and provide gap 46 (space of visible breathable film), which reduces the tension applied to adhesive layer 38 on breathable film 36 and foam ring 33 during removal of paper applicator 39, while still providing sufficient surface and stiffness of the paper applicator to facilitate handling of the assembly during application. Accordingly, in some examples, skin patch 30 preferably includes gap 46 having a width of at least about 3.0 mm, and more preferably about 3.0 to 5.5 mm, e.g., about 4.5 mm, from the periphery of foam ring 33, and paper applicator 39 has a width of at least 3.0 mm, and more preferably about 3.0 to 5.5 mm, e.g., about 4.5 mm. The V-shaped notch may extend on at least 50% of the width of the paper applicator, preferably between 40% and 60%. A distance between two successive slit may be of at least 0.1 mm, such as 0.25 mm or more, preferably between about 0.20 mm and about1.00 mm, more preferably between about 0.5 mm and about 1.00 mm. As may be expected, gap 46 reduces the coverage of paper applicator 39 on patch 30. The lesser coverage of paper applicator 39 results in greater initial and long-term adhesion of patch 30 to the subject's skin because the force required to remove paper applicator 39 is much lower compared to that of paper applicator 18 of patch 10. For example, paper stiffness is detrimental to conformality of breathable film 36 on the skin, and therefore the larger the gap 46 of the breathable film, the easier to maintain on the adhesive on the skin during the removal of paper applicator.

V-shaped notch 40 and slits 44a and 44b illustrated in FIG. 3B also contribute to long-term adhesion, manufacturability and reproducibility of the initial and long-term adhesive force by reducing the amount of caregiver manipulation required to peel paper applicator 39 from exterior surface 37 of breathable film 36. V-shaped notch 40 preferably is formed with an angle α of about 45° so the caregiver can easily grab the edge of the notch. Further, V-shaped notch 40 preferably includes radiused apex 43, to avoid sharp edges that might interfere with packaging. Notch 40 also preferably adjoins slit 44a. Slits 44a and 44b do not extend entirely across the width of paper applicator, thereby ensuring that paper applicator 39 cannot come free during manufacture. The force to tear the slit 44a and 44b is applied from the center to the periphery of the patch, therefore exerting substantially no forces on the edges or corner as was the case in the peel tab design. The orientation of the forces that the paper applicator applies to the present breathable film is well oriented to exert substantially no shear forces on the adhesive edges. In comparison, the peel tab was in prolongation of the breathable film 11 and to remove the peel tab, the force applied induced shear stress and deformed the breathable film. The paper applicator was larger and covered nearly the entire surface of breathable film 11. The present patches do not include a peel tab and incorporate substantially less risk of deforming the breathable film 36 during application. For example, the inventors have observed during *in vitro* testing that the configuration of notch 40 and slits 44a and 44b greatly enhance the ease with which paper applicator 39 can be torn open by a caregiver once patch 30 is adhered to a subject. In this manner, manipulation of the patch once it is applied to the subject is reduced, and the amount of tension applied to the patch during removal of paper applicator 39 is further reduced compared to patch 10.

Substrate 34 may include any suitable material or combination of materials for supporting the active substance and that suitably may be coupled to foam ring 33, e.g., via adhesive on the adjoining surface of the foam ring. In some examples, substrate 34 may include a polymer. Nonlimiting examples of suitable polymers include cellulose plastics (such as cellulose acetate (CA) or cellulose propionate (CP)), polyvinyl chloride (PVC), polypropylene, polystyrene, polyurethane, polycarbonate, polyacrylic (such as poly(methyl methacrylate (PMMA)), polyester, polyethylene (PE), polyethylene terephthalate (PET), or a fluoropolymer (such as polytetrafluoroethylene (PTFE). In nonlimiting examples in which electrospray is used for the deposit of the active substance, substrate 34 may include a conductive layer, e.g., a coating that includes metal particles or metal oxide particles such as titanium oxide, aluminum, or gold, so as to enable positive or negatives charges to be distributed over the backing during the electrospray process and the deposit of the compounds of interest. In one nonlimiting example, substrate 34 of patch 30 include polyethylene terephthalate and a titanium layer coating on its skin-facing surface for use in dissipating electrical current during an electrospray process. The biologically active substance can be bound to the substrate without using an adjuvant and thus the substance retains its reactogenic nature. In some examples, substrate 34 is at least partially transparent, so that the skin surface within the chamber may be visually inspected to monitor for potential adverse effects without removing patch 30. In other examples, substrate 34 is opaque. Breathable film 36 of patch 30 preferably comprises a translucent polyurethane (or silicone) material that is partially coated with a biocompatible, medical grade pressure sensitive adhesive and has a water permeability (moisture vapor transmission rate). Nonlimiting examples of suitable adhesives are provided elsewhere herein.

Advantageously, the circular shape of breathable film 36 avoids right angle corners, such as corners 22 of patch 10, which were observed during in vitro friction tests to be initiation points for detachment of the film. In particular, some test rigs were developed to create mechanical and physical constraints in order to simulate rubbing of a patient's garment on the patch when applied to a simulated subject's skin. A first test rig included a piece of cloth that was rubbed in a reciprocated motion over the patch and simulated skin with reproducible force for a specified number of cycles, thereby enabling multiple patch configurations to be tested. A second test rig was developed to mimic iterative skin elongation constraints for patches applied to simulated skin, with different % of elongation. The patch detachment was assessed according to the number of cycles, necessary to achieve score adhesion, with visual observation. A third test rig with a robotic arm and a scratching finger was developed to mimic human scratching finger applied on the patch applied to simulated skin. Based on such simulations, some of which are further described in the Working Examples below, the present inventors found that substantially removing right angle corners significantly enhanced adhesion of the inventive patches, and that increasing the breathable adhesive surface of the patch significantly enhanced adhesion, with both round and rounded rectangular shapes. This conclusion was confirmed by *in vivo* tests results on human skin with healthy adults as described in the Working Examples.

In nonlimiting examples, whereas patch 10 of FIGS. 1A-1B and 2A-2B was approximately between 30 to 45 mm square for a total patch area around 1100 mm² whereas patch 30 of FIGS. 3 and 4 has a diameter approximately around 45 mm, with a total patch area around 1500-1520 mm², an increase in area of about 30% and a breathable film area increase of about 60%. It will be appreciated that these values are only one example, and that other absolute and relative sizes of breathable film 36 suitably may be used.

Patch 30 differs from patch 10 further in that patch 30 completely eliminates peeling tabs 20a and 20b, removal of which also was observed to reduce initial adhesion during placement of patch 10. In patch 30, breathable film 36 includes no tab of any sort. Instead, release liner 31 includes slit 32 along a chord of the patch. The slit may be asymmetrically located so as to be offset from the portion of the substrate that defines the condensation chamber, so as to inhibit moisture from entering the condensation chamber via the slit. The slit may overlap the foam ring, so as to facilitate peeling the release liner off of the rest of the assembly. For example, the larger part of the release liner may be removed, leaving the smaller part of the release liner attached to the foam ring for use as a handle in appropriately positioning and adhering the assembly to the skin in a manner such as described with reference to FIGS. 26-28. In one nonlimiting example, slit 32 defines width 47, in diagonal and placed on one side and in the middle of the foam ring width (of about 11 mm), as depicted in FIG. 3A. In this manner, release liner 31 may be readily removed from the skin-facing surfaces of breathable film 36 and foam ring 33 by having the caregiver slightly bending the patch to grasp the edges of the release liner exposed by slit 32. The patch without large part release liner then may be applied on the subject's skin, release liner small part could be easily removed and then the patch totally adhered to the skin by pressing firmly on paper applicator 39 in addition to other adhesive surfaces in patch 30, after which paper applicator 39 may be torn open at V-shaped notch 40 and removed from exterior 37 of breathable film 36.

In some examples, patch 30 optionally further comprises label 48, illustratively "DBV" or any other suitable combination of letters and numbers such as shown in FIG. 3A, which may be printed on exterior surface 37 of breathable film 36, or on the interior surface of the breathable film and/or adhesive on the skin facing side of the breathable film, using an inkjet printer, preferably using a food grade ink. In one embodiment, label information may be applied to adhesive film 37 while it is still in a roll form by passing the film through an ink jet printer before the breathable film is cut to shape.

The foregoing features, singly and in combination, have been determined using in vivo and in vitro tests conducted at the direction of the assignee of the present application, significantly to enhance improve manufacturability, ease of manipulation and application, initial and long-term adhesion of patch 30 compared to patch 10, for example as described in the Working Examples further below.

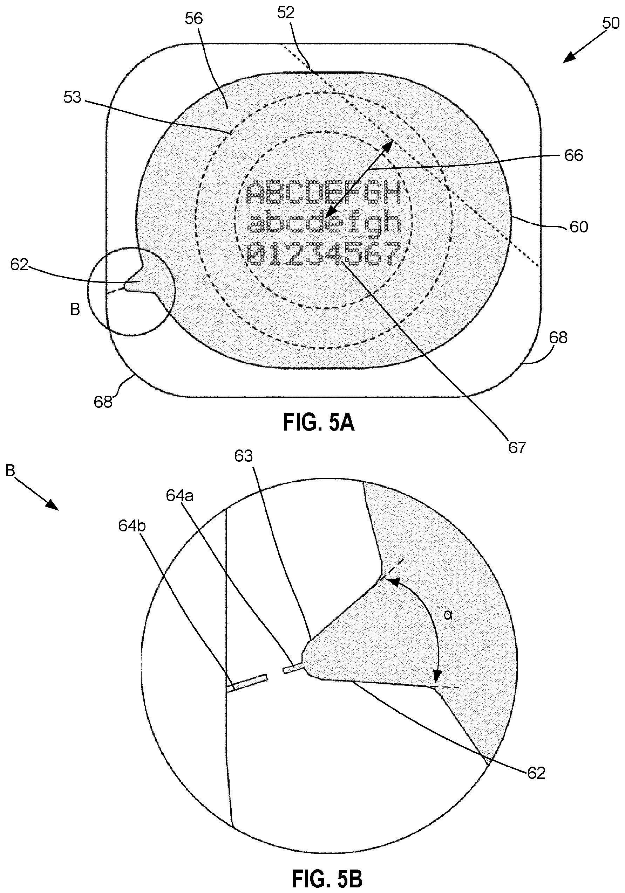

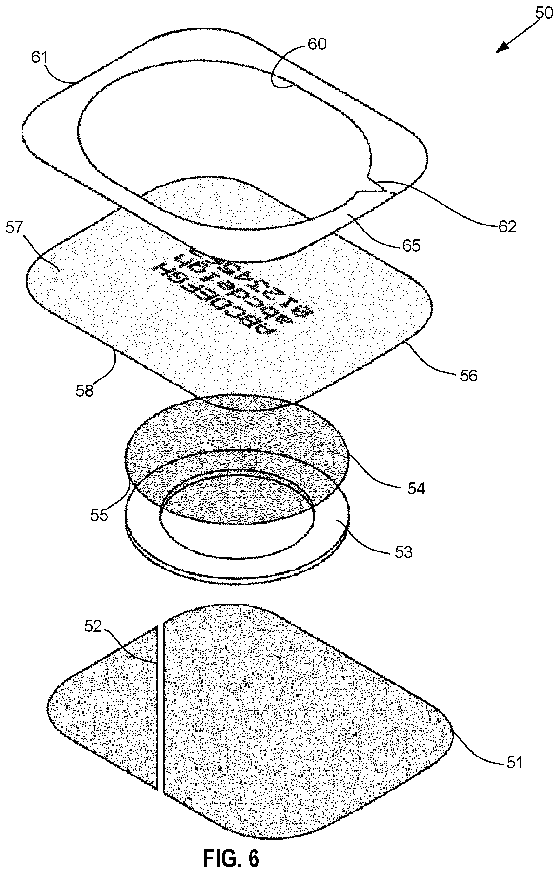

Referring now to FIGS. 5A, 5B and 6, a second embodiment of a patch constructed in accordance with the principles of the present invention is described. Patch 50 is similar in construction to patch 30 of FIGS. 3A-3B and 4, and includes all of the various inventive features discussed above with respect to patch 30. More specifically, patch 50 includes: release liner 51 having slit 52; foam ring 53 having a biocompatible adhesive layer disposed in contact with release liner 51; substrate 54 that holds biologically active substance bound to its skin-facing lower surface 55; breathable film 56 having exterior surface 57 and layer 58 of biocompatible, medical grade pressure sensitive adhesive disposed on its skin-facing surface; and paper applicator 65 disposed on exterior surface 57 of film 56.

Foam ring 53 may be made of similar materials, and may have similar dimensions, as described for foam ring 33. For example, foam ring 53 may have any suitable shape and dimensions to support substrate 54 above a subject's skin, and to create a chamber, when applied to a subject. For example, foam ring 53 may have an external diameter between about 10 mm and about 100 mm, e.g., between about 20 mm and about 50 mm, or between about 20 mm and about 40 mm. Additionally, or alternatively, foam ring 53 may have an internal diameter that is at least about 4 mm less than any of the above external diameters (that is, the width of foam ring 33 may be at least about 2 mm). Additionally, the internal diameter of foam ring 53 may be about 2 mm to 50 mm less than the external diameter, or may be about 2 mm to 20 mm less than the external diameter, or may be about 5 mm to 15 mm less than the external diameter. Foam ring 53 may have any suitable thickness, e.g., about 0.1 mm to about 1 mm, e.g., about 0.5 mm to about 0.9 mm. In one nonlimiting example, foam ring 53 may have an external diameter of about 20-30 mm (e.g., about 26 mm), an internal diameter of about 10-20 mm, (e.g., about 18 mm) and a thickness of about 0.7-0.9 mm (e.g., about 0.8 mm). Foam ring 53 and substrate 54 may have substantially the same outer diameter as one another. For example, these components may be assembled to one another and then die cut together.

In this example, paper applicator 65 has a substantially ring shape with oblong aperture 60 along its interior and rounded rectangular periphery 61 that is co-extensive with breathable film 56. Paper applicator 65 may include V-shaped notch 62 that extends from oval aperture 60 partially towards periphery 61. As shown in detail B in FIG. 5B, V-shaped notch 62 preferably is radially directed, and may have radiused apex 63 that is aligned with slits 64a and 64b to facilitate tearing away of paper applicator 65 once patch 50 is firmly pressed onto the subject's skin. The short axis of aperture 60 may be at least about 1 mm greater than the external diameter of foam ring 53, so as to provide a gap of at least about 1 mm between the foam ring and the paper applicator. The short axis of aperture 60 may, for example, be about 1 mm to about 20 mm greater than the external diameter of foam ring 53, or about 2 mm to about 10 mm greater than the external diameter of foam ring 53, or about 3 mm to about 5 mm greater than the external diameter of foam ring 53. The rounded rectangular periphery 61 of paper applicator may have any suitable dimension that is at least 1 mm greater than the short axis of aperture 60 (that is, paper applicator 65 may have a width of at least 1 mm). For example, paper applicator 61 may have a width of about 1 mm to about 20 mm, or about 2 mm to about 10 mm, or about 3 mm to about 5 mm. In some example, the width of paper applicator 59 is about 10% to about 200% that of the gap between foam ring 53 and paper applicator 61, e.g., about 20% to about 180% that of the gap, or about 50% to about 150% that of the gap, or about 80% to about 120% that of the gap, or about 90% to about 110% of the gap, or about 100% that of the gap. In one nonlimiting example, the width of paper applicator 65 is chosen to cover approximately half of the distance by which breathable film 56 extends beyond the outer edge of foam ring 53 (that is, to have approximately the same width as the gap), and to have a minimal width of 3 mm to provide sufficient stiffness to facilitate handling. In one nonlimiting example, the short axis of oblong aperture 60 has an internal diameter of about 30 mm and a long axis of about 38 mm, thereby providing a smallest gap 66 (see FIG. 5A) of about 3.0 mm to the periphery of foam ring 53. As for patch 30 described above, the width of paper applicator 39 and the size of gap 46 contribute to better control of placing patch 30 during initial placement, and better longer-term adhesion, better manufacturability and uniformity of results compared to patch 10.

As discussed above, during initial clinical trials of patch 10, it was observed that the patch would begin losing adhesion to the subject's skin shortly after initial application. That loss of adhesion is believed largely to result from the removal of paper applicator 18 after patch 10 is applied and the peel tab removed Similarly as for the embodiment of FIGS. 3A-3B and 4, that loss of adhesion is resolved for patch 50 by reducing the width of paper applicator 65, which reduces the tension applied to adhesive layer 58 on breathable film 56 and foam ring 53 during removal of paper applicator 65. For example, D may be the dimension of the patch between the outer periphery of foam ring 53 to the outer periphery of patch 50, and the width of paper applicator 65 may be between about 40-60% of D. In some examples, gap 66 has a smallest width of at least about 3.0 mm between the interior of oval aperture 60 and the periphery of foam ring 53. Although gap 66 reduces the coverage of paper applicator 65 on patch 50, the lesser coverage also reduces the force required to remove paper applicator 65. In this manner, paper applicator 65 of patch 50 provides greater initial and long-term adhesion of patch 50 to the subject's skin compared to that of patch 10.

V-shaped notch 62 and slits 63a and 63b also contribute to long-term adhesion, manufacturability and reproducibility of the initial and long-term adhesive force by reducing the amount of caregiver manipulation required to peel paper applicator 65 from exterior surface 57 of breathable film 56. In particular, V-shaped notch 62 preferably is formed with an angle α of about 45° so the caregiver can easily grab the edge of the notch. Further, V-shaped notch 62 preferably includes radiused apex 63, to avoid sharp edges that might interfere with packaging or with manufacture; for example, a sharp corner may lead to issues with a rotary die cutting tool. Notch 62 also preferably adjoins slit 64a. Slits 64a and 64b do not extend entirely across the width of paper applicator 65, thereby ensuring that the paper applicator cannot come free during manufacture. As observed in in vivo testing, the configuration of notch 62 and slits 64a and 64b greatly enhance the ease with which paper applicator 65 can be removed once patch 50 is applied to a subject. As for the preceding embodiment, manipulation of patch 50 once it is applied to the subject is reduced, with less tension applied to the patch during removal of paper applicator 65 compared to patch 10.

Substrate 54 may include any suitable material or combination of materials for supporting the active substance and that suitably may be coupled to foam ring 53, e.g., via adhesive on the adjoining surface of the foam ring. Nonlimiting examples of materials for use in substrate 54, and examples for applying the active substance thereto, are provided elsewhere herein.

Breathable film 56 of patch 50 preferably comprises a translucent biocompatible polyurethane material coated with a biocompatible, medical grade pressure sensitive adhesive. Nonlimiting examples of suitable adhesives are provided elsewhere herein. Advantageously, the rounded rectangular shape of breathable film 56 avoids right angle corners, such as corners 22 of patch 10. Similarly as observed with the test rig discussed above with respect to patch 30 and in the Working Examples, rounded corners 68 do not become focal points for premature detachment of the film. In addition, patch 50 has increased total adhesive area compared to patch 10 of FIGS. 1A-1B and 2A-2B. For example, whereas patch 10 has a total patch area of about 1156 mm², patch 50 has a total patch area of about 1550 mm², an increase in area of about 34%. It will be appreciated that these values are only one example, and that other sizes of breathable film 56 suitably may be used.

Patch 50 also differs from patch 10 by completely eliminating peeling tabs 20a and 20b, removal of which was observed to reduce initial adhesion during placement of patch 10. For example, patch 50 includes no removable tab on breathable film 56, but instead includes slit 52 in release liner 51, as depicted in FIG. 5A, in diagonal and placed on one side and in the middle of the foam ring width 66 between the slit and the center of substrate 54. This configuration facilitates removal of release liner 51 from the skin-facing surfaces of adhesive film 56 and foam ring 53 by having the caregiver slightly bending the patch to grasp the edges of the release liner exposed by slit 52 in a manner such as described with reference to FIGS. 26-28. Patch 50 then may be positioned on the subject's skin and adhered to the skin by pressing firmly on the entire patch surface, including paper applicator 65. Paper applicator 65 may be torn open at V-shaped notch 62 and removed from exterior 57 of breathable film 56.

In some examples, patch 50 further comprises label 67, illustratively "VIASKIN DBV 712" or any other suitable combination of letters and numbers such as shown in FIG. 5A, which may be printed on the interior or exterior surface of breathable film 56 using an inkjet printer and a food grade ink. Preferably, the label information may be applied to breathable film 57 while it is still in a roll form by passing the film through an ink jet printer before the breathable film is cut to shape.

Similarly as for patch 30, the above-described features of patch 50, singly and in combination, have been determined in vitro and in vivo tests conducted at the direction of the assignee of the present application, significantly to enhance improve manufacturability, ease of manipulation and application, initial and long-term adhesion of patch 50. As will be described below in the Working Examples, several *in vivo* studies were performed on human skin, on healthy adults, conducted to the same conclusion obtained with *in vitro* tests, that means to the superiority of adhesion of the claimed patches as compared to the reference patch described with reference to FIGS. 1A-1B and 2A-2B.

It will be appreciated that the present patches may be manufactured using any suitable combination of operations. In one nonlimiting example, manufacture of patch 30 or patch 50 may include providing (i) a substrate, (ii) a biologically active substance, (iii) a foam ring having a periphery, a first skin-facing surface and a layer of biocompatible adhesive disposed on the first skin-facing surface, (iv) a biocompatible breathable film having an exterior surface, a second skin-facing surface, and a second layer of biocompatible adhesive disposed on the second skin-facing surface and (v) a paper applicator having an aperture with an interior edge. Manufacture of the patch may include affixing the biocompatible foam ring beneath the substrate to form, when applied to a subject's skin, an occlusive chamber. Manufacture of the patch also may include disposing the biologically active substance on the substrate for delivery to a subject's skin via the chamber. Manufacture of the patch also may include applying the biocompatible breathable film concentrically over the substrate and the foam ring. Manufacture of the patch also may include applying the biocompatible paper applicator on the exterior surface of the breathable film so that the aperture is concentric with the foam ring and provides a gap between the interior edge of the paper applicator and the periphery of the foam ring. The paper applicator may be configured to be removed from the exterior surface after the patch is pressed onto a subject's skin.

In a particular embodiment, the method of manufacture of the patch, may comprise the steps of :
- providing (i) a substrate, (ii) a biologically active substance, (iii) a foam ring having a periphery, a first skin-facing surface and a layer of biocompatible adhesive disposed on the first skin-facing surface, (iv) a biocompatible breathable film having an exterior surface, a second skin-facing surface, and a second layer of biocompatible adhesive disposed on the second skin-facing surface and (v) a paper applicator having an aperture with an interior edge;
- affixing the biocompatible foam ring beneath the substrate to form, when applied to a subject's skin, an occlusive chamber;
- disposing the biologically active substance on the substrate for delivery to a subject's skin via the chamber;
- applying the biocompatible breathable film concentrically over the substrate and the foam ring; and
- applying the biocompatible paper applicator on the exterior surface of the breathable film so that the aperture is concentric with the foam ring and provides a gap between the interior edge of the paper applicator and the periphery of the foam ring, the paper applicator configured to be removed from the exterior surface after the patch is pressed onto a subject's skin.

Preferably, the open notch is already present on the paper applicator before being applied on the breathable film.

In some examples, the biologically active substance is bound by electrostatic forces to the substrate. In some examples, the biologically active substance comprises particles.

In some examples, manufacture of the patch further may include creating an open notch, such as a V-shaped notch, in the paper applicator to facilitate removal from the exterior surface of the breathable film. In some examples, the V-shaped notch is radially directed. In some examples, the V-shaped notch has a radiused apex. In some examples, manufacture of the patch further includes creating at least one slit in the paper applicator that is aligned with the open notch to further facilitate removal from the exterior surface of the film.

In some examples, manufacture of the patch includes applying a biocompatible release liner to the first and second adhesive layers. In some examples, manufacture of the patch includes creating a slit in the release liner to facilitate removal from the first and second adhesive layers. In some examples, the slit is located asymmetrically on the release liner and offset from the chamber.

In some examples, providing the breathable film comprises providing a breathable film having a circular shape. In some examples, providing the breathable film comprises providing a breathable film having a rectangular shape with rounded corners. In some examples, providing the breathable film comprises providing a translucent breathable film. In some examples, providing the breathable film comprises providing a breathable film having pre-printed label on the second skin-facing surface.

In some examples, the biologically active substance is selected from an allergen, an antigen and a biologically active polypeptide.

### WORKING EXAMPLES

The following examples are intended to be purely illustrative, and not limiting of the present invention.

### In Vitro Studies: Elongation, Scratching, Friction Tests

*In vitro* tests were developed to mimic and study various constraints and stressors exerted on the system to predict *in vivo* adhesion performance under extreme conditions that could simulate patient use and to identify areas of improvement. The *in vitro* tests were used to study factors contributing to adhesion in the present patches (which may be referred to as the mVP system or mVP patch) as compared to the patches described with reference to FIGS. 1A-1B and 2A-2B (which may be referred to herein as the cVP system or cVP patch).

An elongation bench was used to assess peel resistance through mimicking real-life skin extension conditions. This involved applying the patches to a substrate with properties (e.g., surface tension) similar to human skin and mechanically elongating the patches by various degrees consistent with normal human activity. This also allowed the impact to be assessed of the back position (i.e., curved or straight posture) at application on adhesion performance. The *in vitro* test was used to assess the impact of scratching using a robotic arm with a rubber "finger". The robotic arm was programmed in a variety of ways to mimic scratching related to local system irritation/itching, with the patch adhered to a rubber material with a surface tension similar to human atopic skin.

In addition, various mVP patch designs were compared with the cVP patch design in their capacity to resist friction. A test bench was designed and used to perform back-and- forth movements with varying applied forces, directions and frequencies in order to simulate patch behavior under pressure and friction. These *in vitro* assessments and collaborations allowed for assessment of the impact of mechanical forces and to identify shapes and specific prototypes to advance to *in vivo* analysis.

### In Vivo Study

*In vivo* adhesion performance of multiple mVP patch designs and the cVP patch were assessed in a study that involved 24 hours of application time per system in 48 healthy adult volunteers. In this study, testers applied 4 systems per day per participant over two days (separated by at least 48 hours between Day 1 and Day 2). Each participant wore 2 of the same 4 systems on each day (8 systems in total), with one group of systems applied with the participant in a curved back position, and the other system with a straight back, to assess mVP patches adhesion performance including the impact of posture during system application. Participants were encouraged to participate in any desired physical activities, though it was asked that these activities be similar on the two days; factors such as type of activity and water exposure were documented by the participants. Adhesion, ease of removal, and pain on removal were assessed.

Participants in this study were generally active during their days of application, with 96% reporting physical activity of some sort, and 92% reporting water exposure. Adhesion scores both at application and after 24 hours were generally very good, with very few detachments or disruption of the occlusion chamber. Notably, all the mVP patch designs displayed better adhesion than the cVP patch indifferently with the two postures during system application. The mVP patch designs were less painful to remove, though pain scores were generally low, and the patches were considered not painful to remove.

### Patch Designs

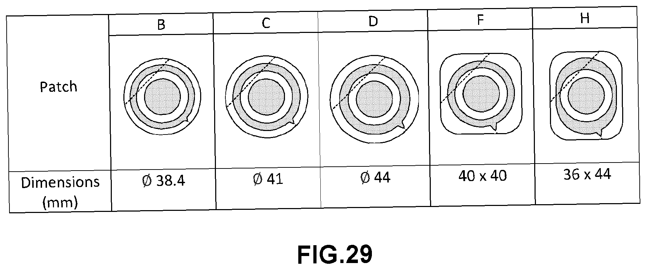

5 mVP systems (round and rectangular rounded) were studied *in vivo,* towards the ultimate goal of advancing one for use in future clinical studies. FIG. 29 illustrates the example patch designs of the study. These *in vivo* assessments to date have been performed in non-peanut-allergic participants at least 18 years of age, with prototypes that have not contained peanut protein.

### CHAMP: Study to Compare Adhesion of cVP patch with mVP patch designs in healthy volunteer subjects

The objective of this study was to compare the adhesion performance of the 5 mVP patch designs versus cVP. This adhesion study was carried out in healthy adult participants (60 randomized), 23% of whom reported a history of atopic dermatitis/eczema, for a planned application duration of 24-hours. Each participant wore all 6 systems, with the location randomized by participant. FIG. 29 presents images of the 5 mVP patches studied: 3 round shaped patches B, C, D, and 2 rectangular rounded patches F, and H (also respectively referred to herein as mVP-B, mVP-C, mVP-D, mVP-F, and mVP-H), along with the dimensions of the PU dressing (breathable film 36 or 56) in contact with skin.

Participants were required to shower and participate in 3 separate 10-minute exercise sessions during the study.

The results of this study confirm that the cVP patch did not perform as well as the mVP patches. Particularly, some cVP patches had lift off or detached of the dressing edges at the time of application (using the previous IFU) relative to mVPs. Additionally, several cVP systems had partial detachment of the foam ring at 20-hours relative, while almost none of the mVP systems. Overall, 15.0% (9/60) of participants experienced adhesion failure with the cVP patch by 20 hours, whereas almost none of participants experienced adhesion failure with the mVP patches by 20 hours. Similar results were obtained at the 24-hour time point. All systems were well tolerated and judged as easy to remove at 24 hours.

Although preferred illustrative embodiments of the present invention are described above, it will be evident to one skilled in the art that various changes and modifications may be made without departing from the invention defined by the claims,

## Claims

1. A patch (30, 50) for delivering a biologically active substance to the skin of a subject, the patch comprising:
a substrate (34, 54);
a biologically active substance disposed on the substrate;
a foam ring (33, 53) having a periphery, a first skin-facing surface and a first layer of adhesive disposed on the first skin-facing surface, the foam ring disposed beneath the substrate to form, when applied to a subject's skin, an occlusive or condensation chamber in which the biologically active substance is located;
a breathable film (36, 56) disposed over the substrate and having an exterior surface (37, 57), a second skin-facing surface opposed to the exterior surface, and a second layer of adhesive (38, 58) disposed on the second skin-facing surface; and
a paper applicator (39, 59, 65) removably disposed on the exterior surface of the breathable film, the paper applicator having an aperture (45, 60) with an interior edge, the paper applicator configured for applying the patch to a subject's skin,
wherein the breathable film covers the substrate and the foam ring, the aperture of the paper applicator is continuous, around and concentric with the foam ring, and the paper applicator has an open notch (40, 62) and at least one slit (44a, 44b, 64a, 64b) aligned with the open notch, the at least one slit extending from an edge of the paper applicator toward the open notch, to facilitate removal from the exterior surface of the breathable film.

2. The patch of claim 1, wherein the open notch opens on the interior edge of the paper applicator.

3. The patch of claim 1 or 2, wherein the open notch is radially directed.

4. The patch of any one of claims 1 to 3, wherein the open notch is a V-shaped notch (40, 62).

5. The patch of claim 4, wherein the V-shaped notch has a radiused apex (43, 63).

6. The patch of claim 4 or 5, wherein the V-shaped notch (40, 62) is formed with an angle α of about 45° +/- 10°.

7. The patch of any one of claims 1 to 6, wherein the paper applicator comprises a plurality of aligned slits, wherein one slit extends from an apex of the open notch.

8. The patch of claim 7, wherein one slit opens on the apex of the open notch.

9. The patch of any one of claims 1 to 8, wherein the open notch is a V-shaped notch (40, 62) and wherein said at least one slit extends from an exterior edge of the paper applicator toward the V-shaped notch to further facilitate removal from the exterior surface of the film.

10. The patch of claim 9, wherein the paper applicator comprises a plurality of slits, wherein one slit (44a, 64a) extends from a radius apex (43, 63) of the V-shaped notch (40, 62).

11. The patch of claim 10, wherein one slit opens on the radius apex of the V-shaped notch.

12. The patch of any one of claims 1 to 11, wherein the internal diameter of the aperture of the paper applicator is about 80% to 95% the greater dimension of said paper applicator.

13. The patch of any one of claims 1 to 12, further comprising a release liner (31, 51) removably disposed on the first and second adhesive layers.

14. The patch of claim 13, wherein the release liner (31, 51) includes a slit (32, 52) to facilitate removal from the first and second adhesive layers.

15. The patch of claim 14, wherein the release liner slit is located asymmetrically, placed on the middle of the foam ring width and offset from the chamber.

16. The patch of any one of claims 1 to 15, wherein the foam ring is circular and the breathable film has a circular shape or a rectangular shape with rounded corners (68).

17. The patch of any one of claims 1 to 16, wherein the substrate is moisture impermeable.

18. The patch of any one of claims 1 to 17, wherein the biologically active substance is selected from an allergen, an antigen and a biologically active polypeptide.

19. The patch of any one of claims 1-18, wherein the biologically active substance is an egg, milk, or peanut allergen.

## Patentansprüche

1. Pflaster (30, 50) zur Abgabe eines biologisch aktiven Stoffes an die Haut eines Patienten, wobei das Pflaster Folgendes umfasst:
ein Substrat (34, 54);
einen auf dem Substrat aufgebrachten biologisch aktiven Stoff;
einen Schaumstoffring (33, 53) mit einem Rand, einer ersten hautzugewandten Oberfläche und einer ersten Klebstoffschicht, die auf der ersten hautzugewandten Oberfläche aufgebracht ist, wobei der Schaumstoffring unter dem Substrat angeordnet ist, um beim Aufbringen auf die Haut des Patienten eine Okklusions- oder Kondensationskammer zu bilden, in der sich der biologisch aktive Stoff befindet;
eine atmungsaktive Folie (36, 56), die über dem Substrat angeordnet ist und die eine Außenfläche (37, 57), eine der Außenfläche gegenüberliegende zweite hautzugewandte Oberfläche und eine zweite Klebstoffschicht (38, 58), die auf der zweiten hautzugewandten Oberfläche angebracht ist, aufweist; und
ein Papierapplikator (39, 59, 65), der abnehmbar auf der Außenfläche der atmungsaktiven Folie angebracht ist, wobei der Papierapplikator eine Öffnung (45, 60) mit einer Innenkante aufweist und der Papierapplikator zum Aufbringen des Pflasters auf die Haut einer Testperson ausgelegt ist,
wobei die atmungsaktive Folie das Substrat und den Schaumstoffring bedeckt, die Öffnung des Papierapplikators durchgehend, umschließend und konzentrisch zum Schaumstoffring ist und der Papierapplikator eine offene Kerbe (40, 62) und mindestens einen Schlitz (44a, 44b, 64a, 64b) aufweist, der mit der offenen Kerbe fluchtet, wobei dieser mindestens eine Schlitz sich von einer Kante des Papierapplikators in Richtung der offenen Kerbe erstreckt, um so das Ablösen von der Außenfläche der atmungsaktiven Folie zu erleichtern.

2. Das Pflaster nach Anspruch 1, wobei die offene Kerbe an der Innenkante des Papierapplikators aufgeht.

3. Das Pflaster nach Anspruch 1 oder 2, wobei die offene Kerbe radial ausgerichtet ist.

4. Das Pflaster nach einem der Ansprüche 1 bis 3, wobei die offene Kerbe eine V-förmige Kerbe (40, 62) ist.

5. Das Pflaster nach Anspruch 4, wobei die V-förmige Kerbe eine abgerundete Spitze (43, 63) aufweist.

6. Das Pflaster nach Anspruch 4 oder 5, wobei die V-förmige Kerbe (40, 62) einen Winkel α von etwa 45° ± 10° aufweist.

7. Das Pflaster nach einem der Ansprüche 1 bis 6, wobei der Papierapplikator mehrere ausgerichtete Schlitze umfasst, wobei sich ein Schlitz von einer Spitze der offenen Kerbe erstreckt.

8. Das Pflaster nach Anspruch 7, wobei sich ein Schlitz an der Spitze der offenen Kerbe öffnet.

9. Das Pflaster nach einem der Ansprüche 1 bis 8, wobei die offene Kerbe eine V-förmige Kerbe (40, 62) ist und wobei sich dieser mindestens eine Schlitz von einer Außenkante des Papierapplikators in Richtung der V-förmigen Kerbe erstreckt, um das Ablösen von der Außenfläche der Folie weiter zu erleichtern.

10. Das Pflaster nach Anspruch 9, wobei der Papierapplikator eine Vielzahl von Schlitzen aufweist, wobei sich ein Schlitz (44a, 64a) von einem Radiusscheitelpunkt (43, 63) der V-förmigen Kerbe (40, 62) erstreckt.

11. Das Pflaster nach Anspruch 10, wobei sich ein Schlitz am Radiusscheitelpunkt der V-förmigen Kerbe öffnet.

12. Das Pflaster nach einem der Ansprüche 1 bis 11, wobei der Innendurchmesser der Öffnung des Papierapplikators etwa 80 % bis 95 % der größeren Abmessung dieses Papierapplikators beträgt.

13. Das Pflaster nach einem der Ansprüche 1 bis 12, ferner umfassend eine Trennfolie (31, 51), die auf der ersten und zweiten Klebeschicht abnehmbar angeordnet ist.

14. Das Pflaster nach Anspruch 13, wobei die Trennfolie (31, 51) einen Schlitz (32, 52) aufweist, um das Ablösen von der ersten und zweiten Klebeschicht zu erleichtern.

15. Das Pflaster nach Anspruch 14, wobei der Schlitz der Trennfolie asymmetrisch angeordnet ist, mittig über der Breite des Schaumstoffrings liegt und von der Kammer versetzt ist.

16. Das Pflaster nach einem der Ansprüche 1 bis 15, wobei der Schaumstoffring kreisförmig ist und die atmungsaktive Folie kreisförmig oder rechteckig mit abgerundeten Ecken (68) ist.

17. Das Pflaster nach einem der Ansprüche 1 bis 16, wobei das Substrat feuchtigkeitsundurchlässig ist.

18. Das Pflaster nach einem der Ansprüche 1 bis 17, wobei der biologisch aktive Stoff ausgewählt ist aus einem Allergen, einem Antigen oder einem biologisch aktiven Polypeptid.

19. Das Pflaster nach einem der Ansprüche 1 bis 18, wobei der biologisch aktive Stoff ein Ei-, Milch- oder Erdnussallergen ist.

## Revendications

1. Patch (30, 50) pour administrer une substance biologiquement active à la peau d'un sujet, le patch comprenant :
un substrat (34, 54) ;
une substance biologiquement active disposée sur le substrat ;
un anneau en mousse (33, 53) ayant une périphérie, une première surface faisant face à la peau et une première couche d'adhésif disposée sur la première surface faisant face à la peau, l'anneau en mousse disposé sous le substrat à former, lorsqu'il est appliqué sur la peau d'un sujet, une chambre d'occlusion ou de condensation dans laquelle est placée la substance biologiquement active ;
un film respirant (36, 56) disposé par-dessus le substrat et ayant une surface extérieure (37, 57), une deuxième surface faisant face à la peau, opposée à la surface extérieure, et une deuxième couche d'adhésif (38, 58) disposée sur la deuxième surface faisant face à la peau ; et
un applicateur papier (39, 59, 65) disposé amoviblement sur la surface extérieure du film respirant, l'applicateur papier ayant une ouverture (45, 60) avec un bord intérieur, l'applicateur papier étant conçu pour appliquer le patch sur la peau d'un sujet,
dans lequel le film respirant couvre le substrat et l'anneau en mousse, l'ouverture de l'applicateur papier est continue, périphérique et concentrique avec l'anneau en mousse, et l'applicateur papier a une encoche ouverte (40, 62) et au moins une fente (44a, 44b, 64a, 64b) alignée avec l'encoche ouverte, la au moins une fente s'étendant depuis un bord de l'applicateur papier vers l'encoche ouverte, afin de faciliter le retrait depuis la surface extérieure du film respirant.

2. Patch selon la revendication 1, dans lequel l'encoche ouverte s'ouvre sur le bord intérieur de l'applicateur papier.

3. Patch selon la revendication 1 ou 2, dans lequel l'encoche ouverte est dirigée radialement.

4. Patch selon l'une quelconque des revendications 1 à 3, dans lequel l'encoche ouverte est une encoche en forme de V (40, 62).

5. Patch selon la revendication 4, dans lequel l'encoche en forme de V a une pointe arrondie (43, 63).

6. Patch selon la revendication 4 ou 5, dans lequel l'encoche en forme de V (40, 62) est formée avec un angle α d'environ 45° +/- 10°.

7. Patch selon l'une quelconque des revendications 1 à 6, dans lequel l'applicateur papier comprend une pluralité de fentes alignées, dans lequel une fente s'étend depuis une pointe de l'encoche ouverte.

8. Patch selon la revendication 7, dans lequel une fente s'ouvre sur la pointe de l'encoche ouverte.

9. Patch selon l'une quelconque des revendications 1 à 8, dans lequel l'encoche ouverte est une encoche en forme de V (40, 62) et dans lequel ladite au moins une fente s'étend depuis un bord extérieur de l'applicateur papier vers l'encoche en forme de V pour faciliter davantage le retrait depuis la surface extérieure du film.

10. Patch selon la revendication 9, dans lequel l'applicateur papier comprend une pluralité de fentes, dans lequel une fente (44a, 64a) s'étend depuis une pointe arrondie (43, 63) de l'encoche en forme de V (40, 62).

11. Patch selon la revendication 10, dans lequel une fente s'ouvre sur la pointe arrondie de l'encoche en forme de V.

12. Patch selon l'une quelconque des revendications 1 à 11, dans lequel le diamètre interne de l'ouverture de l'applicateur papier est d'environ 80 % à 95 % de la dimension la plus grande dudit applicateur papier.

13. Patch selon l'une quelconque des revendications 1 à 12, comprenant en outre un film de protection (31, 51) disposée amoviblement sur les première et deuxième couches adhésives.

14. Patch selon la revendication 13, dans lequel le film de protection détachable (31, 51) comprend une fente (32, 52) pour faciliter le retrait depuis les première et deuxième couches adhésives.

15. Patch selon la revendication 14, dans lequel la fente du film de protection détachable est située asymétriquement, placée au milieu de la largeur de l'anneau en mousse et décalée par rapport à la chambre.

16. Patch selon l'une quelconque des revendications 1 à 15, dans lequel l'anneau en mousse est circulaire et le film respirant a une forme circulaire ou une forme rectangulaire avec des coins arrondis (68).

17. Patch selon l'une quelconque des revendications 1 à 16, dans lequel le substrat est imperméable à l'humidité.

18. Patch selon l'une quelconque des revendications 1 à 17, dans lequel la substance biologiquement active est choisie parmi un allergène, un antigène et un polypeptide biologiquement actif.

19. Patch selon l'une quelconque des revendications 1 à 18, dans lequel la substance biologiquement active est un allergène d'œuf, de lait ou d'arachide.
